# EUROPEAN PATENT SPECIFICATION

(11) **EP 2 174 593 B1**
(45) Date of publication and mention of the grant of the patent: **15.05.2013**
(21) Application number: 08790365.4
(22) Date of filing: 04.08.2008
(51) Int. Cl.: A61B 5/157, A61B 5/151, A61B 5/15

(54) **BLOOD TEST DEVICE AND TEST METHOD**
BLUTTESTVORRICHTUNG UND TESTVERFAHREN
DISPOSITIF DE TEST DE SANG ET PROCÉDÉ DE TEST

(30) Priority: 03.08.2007 JP 2007202488
(43) Date of publication of application: 14.04.2010
(73) Proprietor: Panasonic Corporation, Kadoma-shi Osaka 571-8501 (JP)
(72) Inventor: TAKASHIMA, Tetsuya, Osaka 540-6207 (JP); FUJIWARA, Masaki, Osaka 540-6207 (JP); MIYOSHI, Koji, Osaka 540-6207 (JP)
(74) Representative: Grünecker, Kinkeldey, Stockmair & Schwanhäusser
(86) International application number: PCT/JP2008/002101
(87) International publication number: WO 2009/019854

(56) References cited:
- EP-A1- 1 541 087
- WO-A2-02/078533
- JP-A- 2004 033 376

## Description

### Technical Field

The present invention relates to a blood test apparatus and a test method using the blood test apparatus.

Diabetes patients need to measure their blood sugar level on a regular basis and inject insulin based on the measured blood sugar level to maintain a normal blood sugar level. To maintain this normal blood sugar level, diabetes patients need to measure the blood sugar level on a regular basis. Therefore, patients puncture the skin of their fingers and so forth by using a blood test apparatus, sample a small amount of blood exuding from the skin and analyze the components, such as blood sugar level, based on the sampled blood.

Conventionally, the blood test apparatus disclosed in Patent Document 1 and Patent Document 2 have been known.

For example, steps of a blood test using the blood test apparatus disclosed in Patent Document 1 are as follows. First, the patient touches the blood test apparatus with a finger of one hand (e.g. the index finger of the left hand), pushing a puncturing button of the blood test apparatus by the other hand (e.g. the right hand) and ejecting a puncture needle from a lancet. By this means, the blood test apparatus punctures the skin of the finger touching the blood test apparatus and forms a droplet of blood on the surface of the skin. Next, the patient brings one of blood sensors stacked and stored in a cartridge installed in the blood test apparatus close to the puncturing position to make the sensor touch the blood. By this means, the blood test apparatus analyzes the components of the blood taken into the blood sensor.

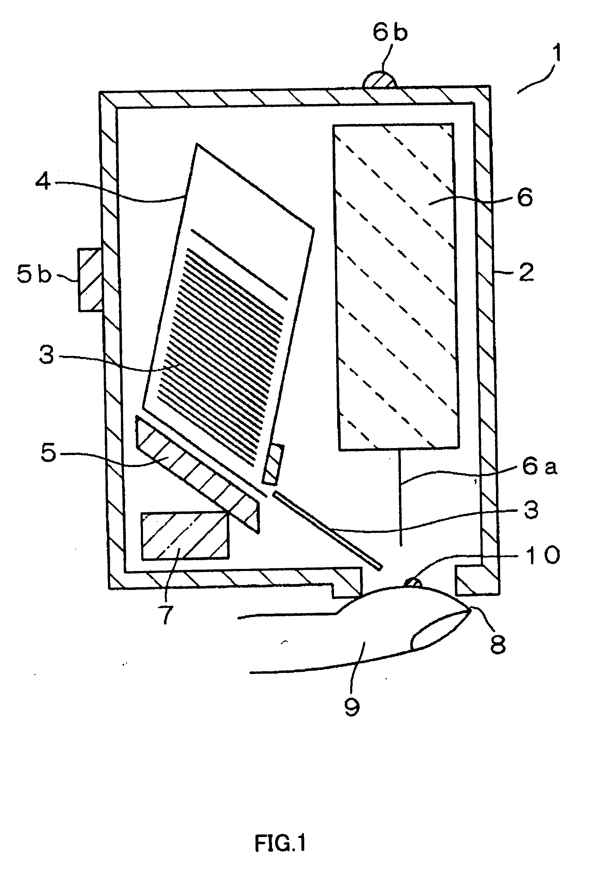

Here, a general configuration of a conventional blood test apparatus will be described. FIG.1 is a cross sectional view showing the configuration of a conventional blood test apparatus.

In FIG.1, blood test apparatus 1 has a substantially rectangular solid shape and includes housing 2 having puncturing section 8 where blood sensor 3 is mounted, and further includes, inside this housing 2, cartridge 4 in which blood sensors are stacked and stored; conveying means 5 that conveys blood sensors stored in cartridge 4 from a sensor outlet to puncturing section 8 one by one; needle puncturing means 6 that faces puncturing section 8 and punctures skin 9 with puncture needle 6a; and electrical circuit section 7 electrically connected with blood sensor 3 that has been conveyed to puncturing section 8. In addition, puncturing button 6b that ejects puncture needle 6a and conveying button 5b that drives conveying means 5, are provided on the surface of housing 2.

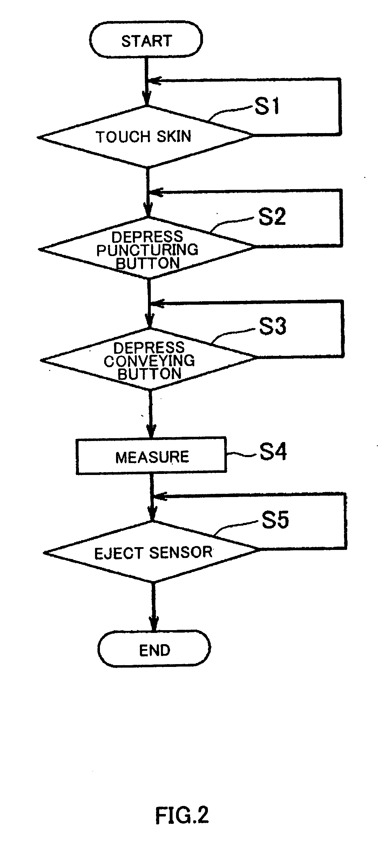

FIG.2 is a flowchart showing a test method using the above-described blood test apparatus 1.

First, in step S1, the user holds blood test apparatus 1 by the right hand and touches puncturing section 8 with skin 9 of the index finger of the left hand. In step S2, the user presses puncturing button 6b by the thumb of the right hand and makes puncturing means 6 eject puncture needle 6a to puncture skin 9 of the index finger of the left hand. Blood 10 exudes on the surface of skin 9 by puncturing skin 9.

In step S3, the user waits until blood 10 sufficiently exudes, and then presses conveying button 5b by the middle finger of the right hand while the positional relationship between puncturing section 8 and the index finger of the left hand is kept constant. When conveying button 5 is pressed, conveying means 5 drives, so that blood sensor 3 at the bottom stored in sensor cartridge 4 is conveyed to puncturing section 8. Then, blood 10 exuding from skin 9 is taken into blood sensor 3.

In step 4, the property of blood 10 taken into blood sensor 3 is measured by electrical circuit section 7 electrically connected to this blood sensor 3, and when the measurement is completed, the step moves to step 5. In step 5, blood sensor 3 stained with blood 10 is removed from puncturing section 8 and discarded.
Patent Document 1: Published Japanese Translation of PCT application No.2004-519302 = WO 02078533 (D1).
Patent Document 2: Published Japanese Translation of PCT application No.2003-524496

### Disclosure of Invention

### Problems to be Solved by the Invention

However, with such conventional blood test apparatus 1, when blood sensor 3 stained with blood 10 is removed from puncturing section 8 and discarded, blood 10 applied to blood sensor 3 contacts puncturing section 8 and stains puncturing section 8, so that puncturing section 8 might be unsanitary.

The present invention has been made in view of the above-described problem. It is therefore an object of the invention is to provide a blood test apparatus that, after blood is measured using the blood sensor held by the holding part of the puncturing section and so forth, the used blood sensor can be discarded without staining the holding part.

### Means for Solving the Problem

The blood test apparatus according to the present invention that takes blood exuding from punctured skin into a blood sensor to analyze components of the blood, the blood test apparatus has a configuration including: a first holder and a second holder that sandwich the blood sensor, at least one of the first holder and the second holder is provided movably in a direction in contact with the other holder and in a direction to part from the other holder; and a gap defining section that moves at least the one holder, so that the first holder and the second holder are placed apart from one another, that supports the blood sensor apart from both the first holder and the second holder, and that defines gaps between the blood sensor and the first holder and between the blood sensor and the second holder.

The test method according to the present invention is a blood test method, using the above-described blood test apparatus, for taking blood exuding from punctured skin into a blood sensor to analyze components of the blood, includes: taking the blood into the blood sensor while the blood sensor is sandwiched between a first holder and a second holder; and forming gaps between the blood sensor and the first holder and between the blood senor and the second holder by defining a space between the blood sensor into which the blood is taken and both the first holder and the second holder sandwiching the blood sensor, by a gap defining section.

### Advantageous Effects of Invention

According to the present invention, after blood is measured using the blood sensor held by the holding part of the puncturing section and so forth, the used blood sensor can be discarded without staining the holding part and the puncturing section can be kept clean after measurement.

### Brief Description of Drawings

FIG.1 is a cross sectional view of a conventional blood test apparatus;
FIG.2 is a flowchart of a test method of the conventional blood test apparatus;
FIG.3 is a cross sectional view of a blood test apparatus according to embodiment 1 of the present invention;
FIG.4 is a cross sectional view of the blood test apparatus in a state in which a cover is open according to embodiment 1 of the present invention;
FIG.5 is an enlarged cross sectional view of a puncturing section and a gap defining section of the blood test apparatus shown in FIG.3;
FIG.6 is a perspective view of the puncturing section and the gap defining section of the blood test apparatus as shown in FIG.3;
FIG.7 is a drawing explaining the gap defining section of the blood test apparatus according to embodiment 1 of the present invention;
FIG.8A is a cross sectional view of the first state of the puncturing section for explaining operation of the gap defining section of the blood test apparatus according to embodiment 1;
FIG.8B is a cross sectional view of the second state of the puncturing section for explaining operation of the gap defining section of the blood test apparatus according to embodiment 1;
FIG.8C is a cross sectional view of the third state of the puncturing section for explaining operation of the gap defining section of the blood test apparatus according to embodiment 1 of;
FIG.9 is a cross sectional view of the blood sensor stored in a cartridge of the blood test apparatus according to embodiment 1;
FIG.10 is a perspective plane view of the sensor of the blood test apparatus according to embodiment 1;
FIG.11 is an external perspective view of the sensor of the blood test apparatus according to embodiment 1;
FIG.12 is a side view of a first holder constituting the puncturing section;
FIG.13 is a perspective view of the first holder from the bottom of the blood test apparatus according to embodiment 1;
FIG.14 is a cross sectional view of a second holder constituting the puncturing section of the blood test apparatus according to embodiment 1 of the present invention;
FIG.15 is a cross sectional view showing an alternative example of the puncturing section of the blood test apparatus according to embodiment 1;
FIG.16 is a perspective view of the first holder constituting an alternative example of the puncturing section of the blood test apparatus according to embodiment 1;
FIG.17 is a perspective view of the second holder constituting an alternative example of the puncturing section of the blood test apparatus according to embodiment 1;
FIG.18 is a cross sectional view explaining operation of the gap defining section for the alternative example of the puncturing section of the blood test apparatus according to embodiment 1;
FIG.19 is a perspective plane view of the cartridge mounted in the blood test apparatus according to embodiment 1;
FIG.20 is a cross sectional view of a laser unit according to embodiment 1;
FIG.21 is a cross sectional view of the laser unit and its nearby primary parts when measurement is performed according to embodiment 1;
FIG.22 is a block diagram of an electrical circuit section and its neighborhood according to embodiment 1;
FIG.23 is a flow chart of the test method according to embodiment 1;
FIG.24 is a perspective view of a first holder constituting a puncturing section according to embodiment 2;
FIG.25 is a perspective view of the second holder constituting the puncturing section according to embodiment 2;
FIG.26A is a cross sectional view showing the first state for explaining operation of a gap defining section according to embodiment 2;
FIG.26B is a cross sectional view showing the second state for explaining operation of the gap defining section according to embodiment 2;
FIG.26C is a cross sectional view showing the third state for explaining operation of the gap defining section according to embodiment 2;
FIG.27 is a perspective view of a sensor receiving member constituting the gap defining section according to embodiment 3;
FIG.28 is a perspective view of the sensor receiving member in which a sensor is inserted according to embodiment 3;
FIG.29 is a drawing of the sensor receiving member shown in FIG.24 viewed from the back side according to embodiment 3;
FIG.30 is a cross sectional view of the first holder where the sensor receiving member is suspended according to embodiment 3;
FIG.31A is a cross sectional view showing the first state in which the cover is open and the puncturing section is in the puncturing possible state, in the blood test apparatus according to embodiment 3;
FIG.31B is a cross sectional view showing the second state in which puncturing operation by the laser unit is completed and the cover is closed, in the blood test apparatus according to embodiment 3;
FIG.31C is a cross sectional view showing the third state in which the sensor is being removed, in the blood test apparatus according to embodiment 3.

### Best Mode for Carrying Out the Invention

Now, embodiments of the present invention will be described in detail below with reference to the accompanying drawings.

### (Embodiment 1)

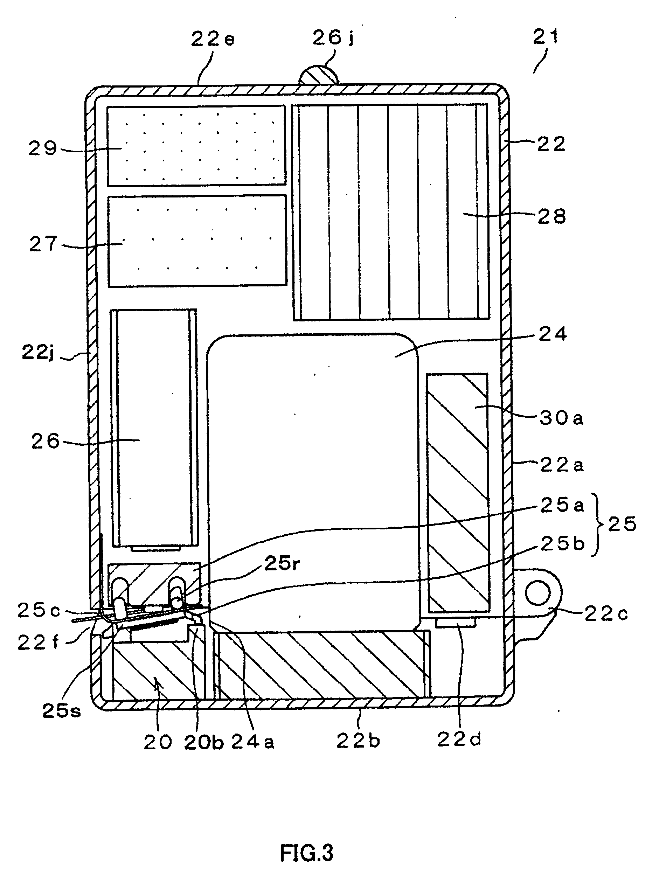

FIG.3 is a cross sectional view showing the configuration of primary parts of the blood test apparatus according to embodiment 1. First, the summary of the blood test apparatus according to embodiment 1 will be described.

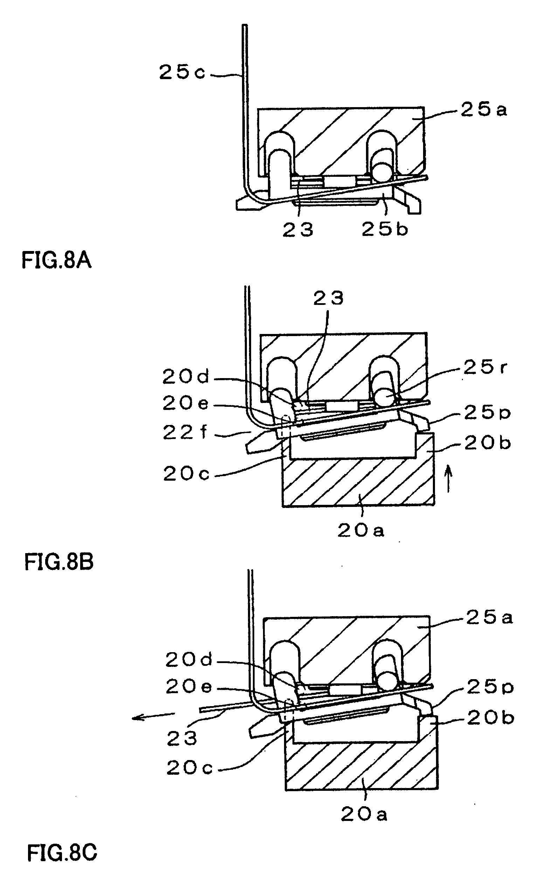

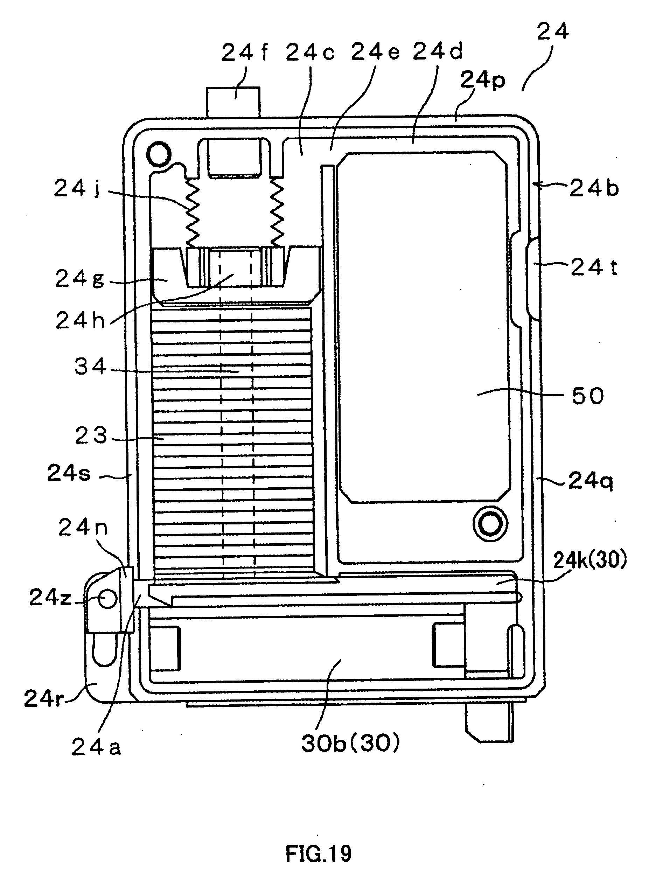

Blood test apparatus 21 has, inside housing 22, cartridge 24 stacks and stores blood sensors 23 (see FIG.8 to FIG.10), puncturing section 25, laser unit (puncturing means) 26, electrical circuit section 27, negative pressure means 28, battery 29, and conveying section 30a that drives conveying means 30 (see FIG.19).

Housing 22 includes; housing body 22a that is made of resin and so forth, has a substantially rectangular solid shape and opens in its one side (the bottom side); and cover 22b that is pivotally mounted on housing body 22a via supporting point 22c and opens and closes the opening of housing body 22a.

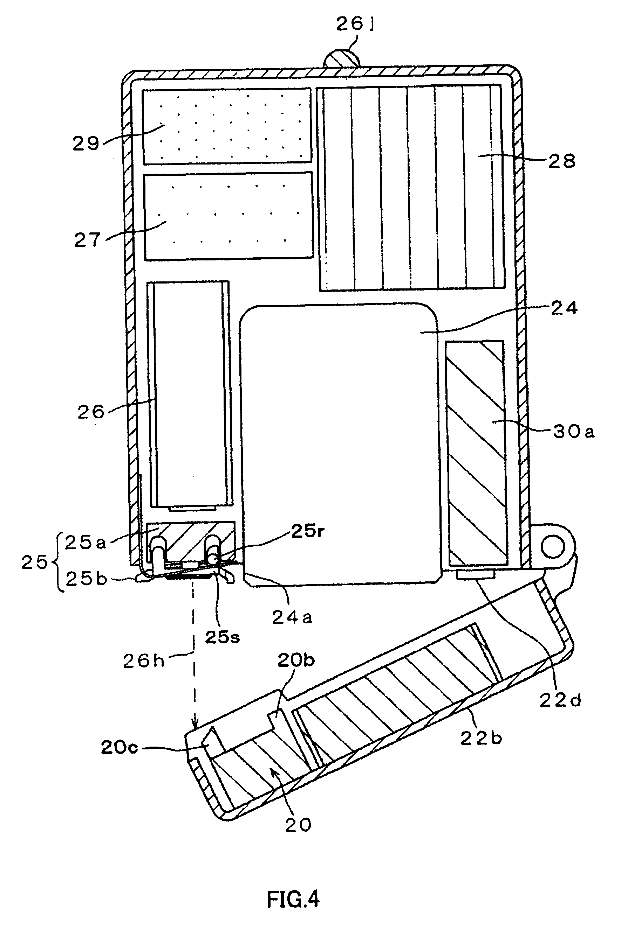

FIG.4 is a cross sectional view of a state in which the cover is open in the blood test apparatus according to embodiment 1.

As shown in FIG.3, housing body 22a is covered in its one side with cover 22b and has puncturing section 25 at the corner of the circumference of this covered opening. Cover 22b is opened from housing body 22a (see FIG4), so that puncturing section 25 located in housing body 22a is exposed outside, which allows to puncture skin and so forth of the user. That is, puncturing section 25 located in housing body 22a is provided in housing body 22a so as to face the opening of housing body 22a, and when cover 22b is opened (see FIG.4), puncturing section 25 located in housing body 22a is exposed outside. Here, puncturing section 25 may be located to face the opening of housing body 22a. For example, puncturing section 25 may be provided to front the opening of housing body 22a, or may be provided to contact the opening of housing body 22a. Moreover, puncturing section 25 may be provided in the housing body 22a, in the vicinity of the opening of housing body 22a.

In addition, on one side wall of housing 22 (left side wall in FIG.3), outlet 22f that ejects used blood sensor 23 from inside housing 22 to outside housing 22 is formed. Here, outlet 22f is formed by cutting out one side surface, which is on the same plane as and in contact with the bottom end of one side wall part 22j in housing body 22a in the vicinity of puncturing section 25 when cover 22b is closed.

Cover 22b rests in a state in which housing 22 is closed shown in FIG.3, that is, in a state in which the end opposite to supporting point 22c (here, supporting point 22 is located in the base end side) is in contact with housing body 22a and covers puncturing section 25. In addition, cover 22b rests in two levels, that is, in the first resting position where cover 22b opens with respect to housing body 22a shown in FIG.4B at about 30 degrees and in the second resting position where cover 22b is open with respect to housing body 22a at about 90 degrees. In each resting position, puncturing section 25 is exposed outside.

In addition, the opening and closing of cover 22b is detected by cover detecting sensor 22d provided at one end (bottom end) of housing body 22a covered with cover 22b. Here, although the present embodiment employs a mechanical switch as cover detecting sensor 22d, cover detecting senor 22d is not limited to this and may be one that detects electrical conduction. In addition, cover detecting sensor 22d may be an optical sensor using light emitting diode and photo-transistor, or may be a magnetic sensor.

Puncturing button 26j is provided on the other side of housing 22 (specifically, the top wall; the upward direction of FIG.3 and FIG.4).

In blood test apparatus 21, laser light 26h (see FIG.4) as a perforatorium is emitted from laser unit 26 by pressing puncturing button 26j while skin 9 touches puncturing section 25 in a state in which cover 22b is open, and puncturing section 25 punctures skin 9. Here, at this time, cover 22b is open at about 30 degrees in the first resting position, so that laser light 26h does not leak outside even if there is not skin 9, and therefore safety can be assured.

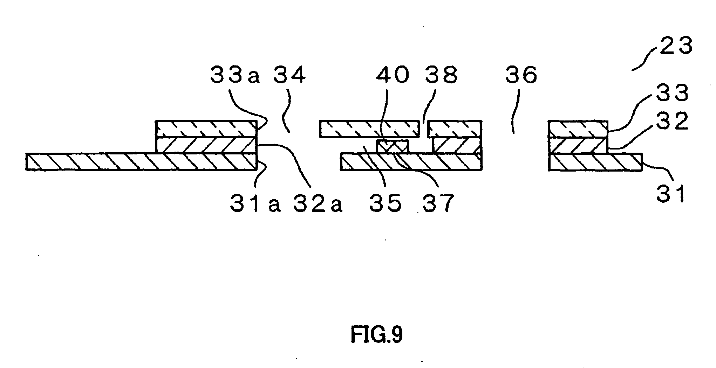

Cartridge 24 is removably mounted in housing body 22a, and stacks and stores blood sensors 23 (see FIG.9 to FIG.11). This cartridge 24 can be easily inserted in and removed from housing 22 by resting cover 22b in the second resting position in which cover 22b is open at about 90 degrees.

Cartridge 24 mounted in housing body 22a is located adjacent to puncturing section 25. In addition, sensor outlet 24a is formed on the lower part of cartridge 24 and in the position facing puncturing section 25. By this means, blood sensor 23 are conveyed through this sensor outlet 24a and is mounted in puncturing section 25.

Blood sensors 23 in cartridge 24 are conveyed through sensor outlet 24a by conveying means 30 (see FIG.19) and are mounted in puncturing section 25. Here, conveying means 30 is provided in cartridge 24, driven by drive section 30a and conveys blood sensor 23 stored in cartridge 24 at the bottom to puncturing section 25. Here, the inner configuration of cartridge 24 will be described in detail later.

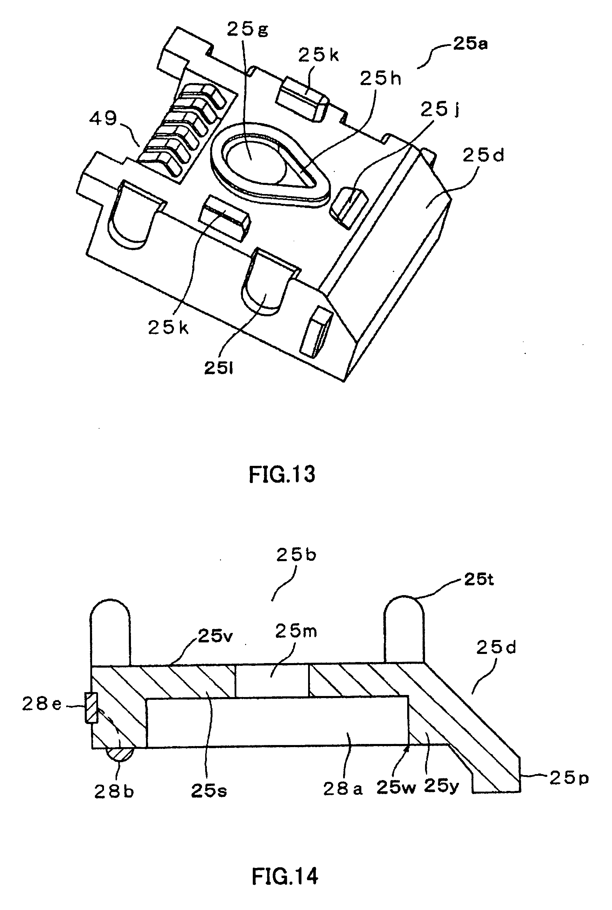

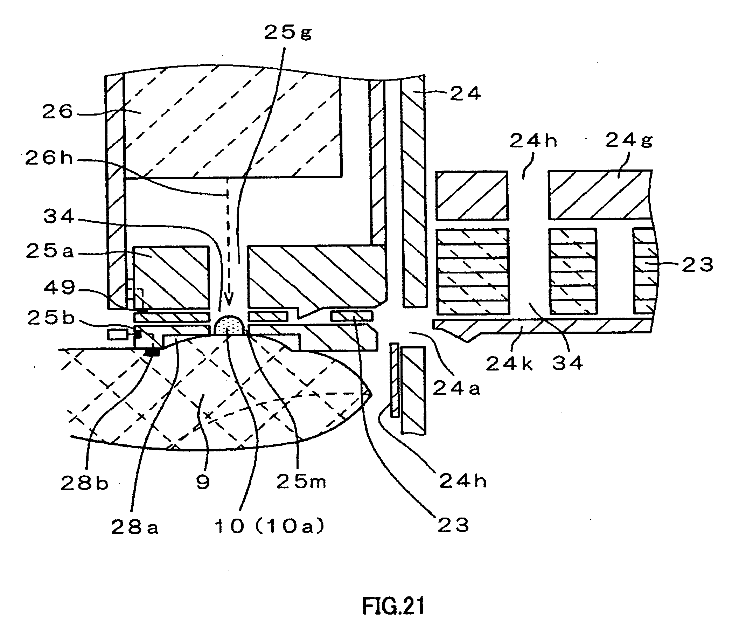

As shown in FIG.4, puncturing section 25 is provided so as to be exposed outside from housing body 22a when cover 22b opens, and has first holder 25a and second holder 25b that sandwich blood sensor 23. Puncturing section 25 punctures the skin in contact with puncturing section 25 and stores blood exuding on the surface of the skin in blood sensor 23 sandwiched and held between first holder 25a and second holder 25b. Here, negative pressure chamber 28a (see FIG.14) coupled to negative pressure means 28 through a negative pressure path (not shown) is formed under puncturing section 25. In addition, skin detecting sensor 28b (see FIG.14) that detects skin 9 (see FIG.21 described later) is provided adjacent to this negative pressure chamber 28a. Here, since the skin touches the surface (under surface) in one side of second holder 25b, which is exposed outside, skin detecting sensor 28b is provided on the under surface of second holder 25b.

The surface where first holder 25a and second holder 25b contact one another is formed such that sensor outlet 24a and blood sensor 23 stacked at the bottom among blood sensors 23 stacked and stored in cartridge 24 are in alignment with this surface. Blood sensors 23 stacked and stored in cartridge 24 are separated, and one blood sensor 23 conveyed from sensor outlet 24a is inserted between those first holder 25a and second holder 25b.

Gap defining section (pushing-up section) 20 that defines a gap between first holder 25a and blood sensor 23 and a gap between blood sensor 23 and second holder 25b when second holder 25b is placed apart from second holder 25b is provided in the vicinity of first holder 25a and second holder 25b.

In the state in which cover 22b is closed as shown in FIG.3, first holder 25a and second holder 25b are located by gap defining section 20 in positions spaced from one another, and also are spaced from blood sensor 23 sandwiched therebetween. That is, in the state in which cover 22b is closed, gap defining section 20 operates to make a space between first holder 25a and second holder 25b, so that gaps are defined between first holder 25a and blood sensor 23 and between second holder 25b and blood sensor 23, respectively.

These gaps make it possible to prevent blood 10 applied to blood sensor 23 in puncturing from staining first holder 25a and second holder 25b, so that first holder 25a and second holder 25b can be kept clean after puncturing.

Here, gap defining section 20 will be described in detail along with explanation of puncturing section 25.

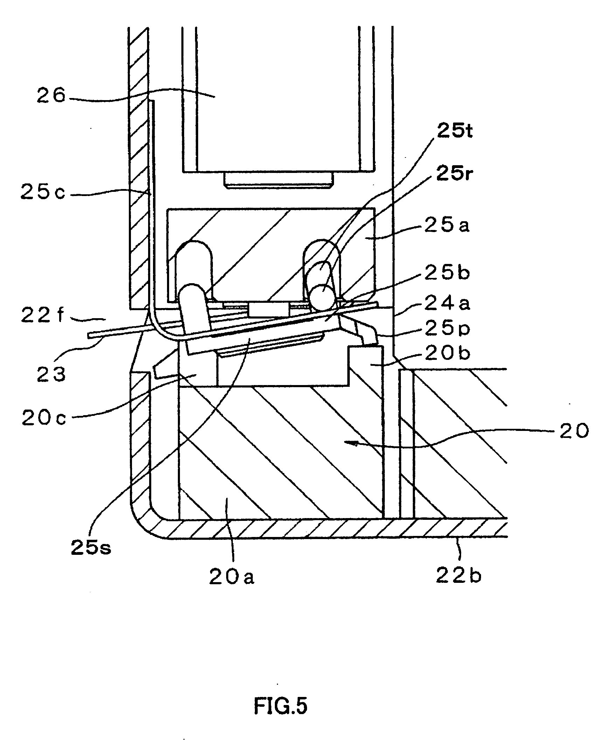

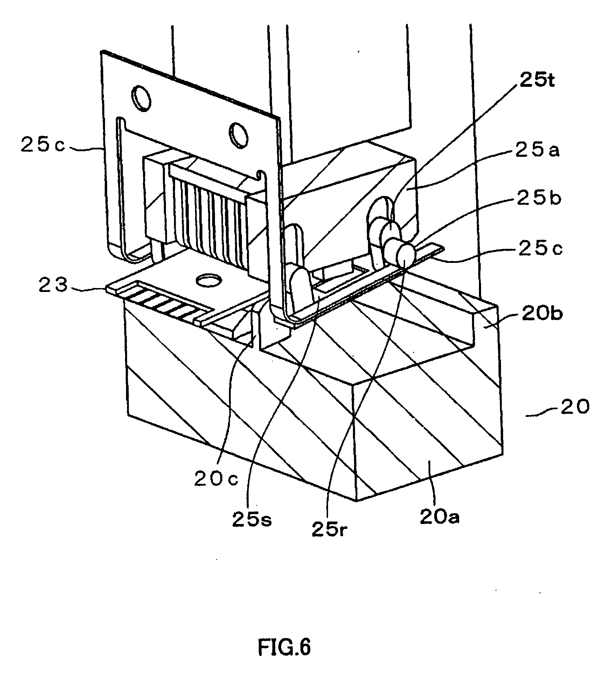

FIG.5 is an enlarged cross sectional view of the puncturing section and the gap defining section of the blood test apparatus shown in FIG.3. FIG.6 is a perspective view of the puncturing section and gap defining section 20 shown in FIG.5 Here, FIG.5 and FIG.6 show the blood test apparatus in the state in which the cover is closed after puncturing.

First holder 25a and second holder 25b shown in FIG.3 to FIG.6 face one another and are provided and move in the contacting direction and in the spacing direction. Here, first holder 25a is fixed to the side surface of housing body 22a, and second holder 25b is attached to housing body 22b such that second holder 25b can rotate around spindle 25r and move in the direction to contact first holder 25a and in the direction to part from first holder 25a. Here, second holder 25b in the sensor outlet 24a side is biased toward first holder 25a by a bias member (leaf spring 25c). By this means, the whole of second holder 25b is biased toward first holder 25a.

In the present embodiment, spindle 25r is provided in second holder 25b in the sensor outlet 24 side and parallel to supporting point 22c.

Blood sensor 23 is sandwiched between holder body 25s of second sensor 25b and first holder 25a. Holder body 25s of second sensor 25b extends from the vicinity of sensor outlet 24a toward the ejecting direction of blood sensor 23.

As shown in FIG.6, spindle 25r is formed so as to be perpendicular to guide pieces 25t rising from one end (referred to as "base end" for convenience) in the sensor outlet 24a side at both rims of holder body 25s.

The end of leaf spring 25c rising with a slope from the inner surface of one side wall 22j (see FIG.3) is engaged with spindle 25r. This leaf spring 25c allows second holder 25b to be biased toward first holder 25a in the base end side (sensor outlet 24a side).

Second holder 25b swings with respect to first holder 25a around spindle 25r and sandwiches blood sensor 23 with first holder 25a at the center part between the other end (referred to as "leading end" for convenience) and spindle 25r.

In addition, as shown in FIG. 5, tongue piece 25p is provided in holder body 25s such that tongue piece 25p projects from the base end from which guide part 25t rises to sensor outlet 24a and bends downward.

In the state in which cover 22b is closed as shown in FIG.3, FIG.5 and FIG.6, tongue piece 25p is pressed upward by pressing projection 20b of gap defining section 20, which rises from cover 22b. By this means, holder body 25s of second holder 25b is arranged in a position apart from first holder 25a such that the spacing distance increases from the base end toward the leading end. That is, the area between first holder 25a and second holder 25b is enlarged toward outlet 22f.

In the state in which cover 22b is closed as shown in FIG.3, FIG.5 and FIG.6, pressing projection 20b projects from pushing-up section body 20a provided in the location of cover 22b, which faces puncturing section 25 to the housing body 22a side, specifically, the puncturing section side 25 side.

Pressing projection 20b of pushing-up section body 20a has a projection-like shape rising from the location in the sensor outlet 24a side, which faces tongue piece 25p of second holder 25b, and its edge includes a rectangular surface having longer sides in the direction of spindle 25r. The width of this edge surface (the length in the direction of conveyance of blood sensor 23) is larger than tongue piece 20p so as to surely press tongue piece 25p when cover 22b is closed.

In addition, support pawl 20c is formed in the location of pushing-up section body 20a in the outlet 22f side, which rises toward puncturing section 25 and supports blood sensor 23 by abutting on blood sensor 23 from below when second holder 25b is placed apart from first holder 25a

Those pushing-up section body 20a, pressing projection 20b and support pawl 20c constitutes the pushing-up section as gap defining section 20.

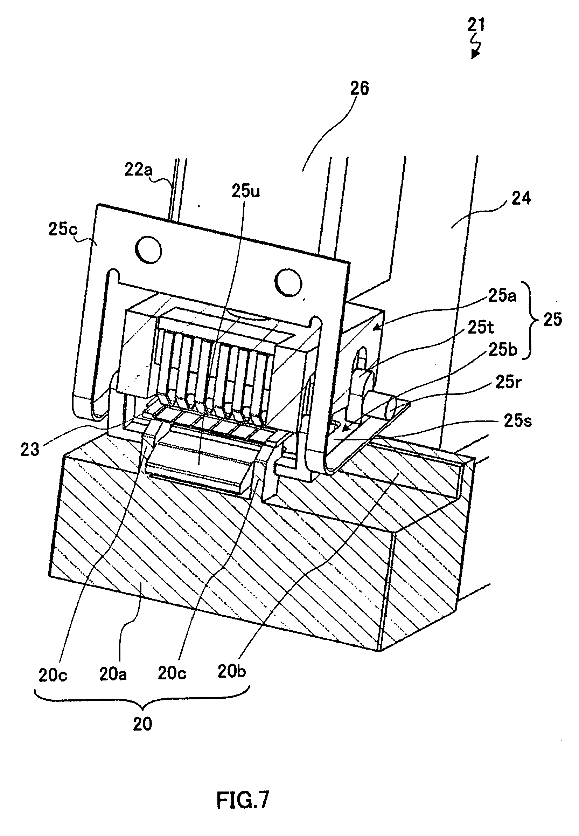

FIG.7 is a drawing explaining the gap defining section of the blood test apparatus according to embodiment 1 of the present invention, and is a perspective view showing the puncturing section and the gap defining section immediately after the cover is closed after puncturing.

As shown in FIG.7, the positional relationship between pawls 20c and blood sensor 23 is established such that supporting pawls 20c abut on blood sensor 23 at both rims of blood sensor 23 perpendicular to the conveying direction of blood sensor 23 and support blood sensor 23 in the positions apart from the circumference of storing section 34 (see FIG.9, FIG.10 and FIG. 11) formed at the center of blood sensor 23.

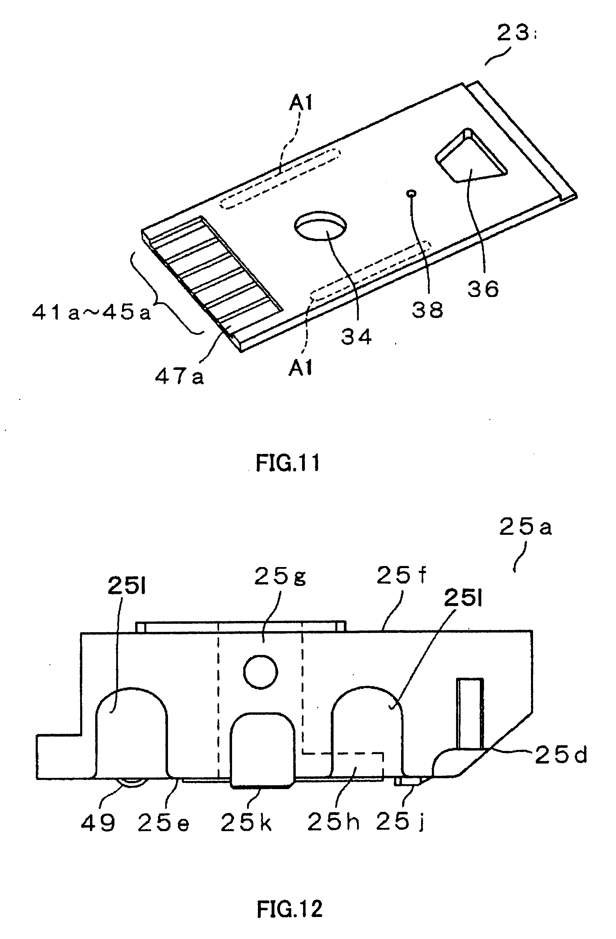

That is, assuming that the surface of blood sensor 23, on which second holder 25b is located is the back surface, supporting pawls 20c support a part of the circumference (region Al s shown in FIG.11) or the circumference of the back surface of blood sensor 23.

Here, as shown in FIG.7, supporting pawls 20c are formed to project toward first holder 25a through cutout parts resulting from cutting out both sides of center part 25u, at the leading edge of second holder 25b spaced from first holder 25a when cover 22b is closed. As described above, the tips of the members projecting from holder body 25s of second holder 25b toward first holder 25a abut on the both side rims of the back surface of blood sensor 23, so that blood sensor 23 itself is arranged to be spaced from second holder 25b.

Here, since first holder 25a is fixed to housing body 22a and also laser unit 26 is fixed to housing body 22a, the distance between laser unit 26 to second holder 25b stays constant. Therefore, the puncturing depth can be the set value. This result can be obtained by another configuration fixing second holder 25b and biasing first holder 25a toward second holder 25b by a bias member (corresponding to spring 25c)

Laser unit 26 is arranged to face puncturing section 25 in housing body 22a. Here, laser unit 26 is mounted in the upper part (above) of puncturing section 25, which is exposed outside when cover 22b is open and punctures the skin of the user in contact with puncturing section 25 by emitting laser light.

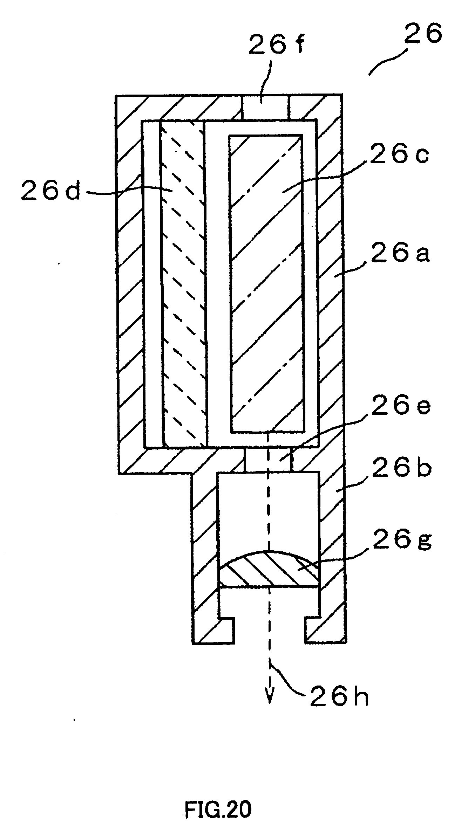

Here, when laser light is emitted, cover 22b is open at about 30 degrees in the first resting position, so that laser light 26h (see FIG.4) does not leak outside, so that safety is ensured. In addition, cover 22b is closed when blood test apparatus 21 is not used, so that laser light does not leak outside even if the user and so forth presses puncturing button 26j by accident and laser light is emitted accidentally, and therefore safety can be ensured. Moreover, cover 22b is closed when blood test apparatus 21 is not used, so that the user does not touch skin detecting sensor 28b (see FIG.14 and FIG.20) by accident, therefore it is possible to prevent laser light from emitting when the blood test apparatus 21 is not used.

Here, although the puncturing apparatus employs laser unit 26 as a puncturing means in the present embodiment, the puncturing means is not limited to this and needle puncturing device using a puncture needle is applicable. In this case, consumption of battery 29 is reduced.

Electrical circuit section 27 (see FIG.3 and FIG.4) is arranged between laser unit 26 and the other side 22e and is electrically connected with blood sensor 23, laser unit 26, negative pressure means 28 and so forth and skin detecting sensor 28b. Electrical circuit section 27 commands laser unit 26 to perform puncturing (here, command to emit laser light). In addition, electrical circuit section 27 analyzes the components of blood taken into blood sensor 23. Moreover, electrical circuit 27 commands negative pressure means 28 to produce a negative pressure at a predetermined timing.

Negative pressure means 28 shown in FIG.3 and FIG.4 applies a negative pressure to the vicinity of puncturing section 25 and to inside cartridge 24 according to the command from electrical circuit section 27. The negative pressure applied to puncturing section 24 allows the skin to be located on the puncturing section 25 and to position the surface of the skin in the reference position to determine the puncturing depth. Here, this reference position is the focal position of laser light if the perforatorium for puncturing skin is laser light, or, a position where the sufficient puncturing depth can be assured if the perforatorium is a puncture needle.

Negative pressure means 28 is composed of a pump and a solenoid valve (not shown). A negative pressure produced by negative pressure means 28 is introduced into negative pressure chamber 28a (see FIG.14) of puncturing section 25 through the negative pressure path in housing body 22a. The negative pressure is also introduced into cartridge 24 through the negative pressure path in housing body 22a. Those of supply of a negative pressure between negative pressure chamber 28a of puncturing section 25 and cartridge 24 can be switched by the valve. Negative pressure means 28 supplies a negative pressure to each of negative pressure path 24f (see FIG.19) and negative pressure chamber 28a (see FIG.1 and FIG.21). Electrical circuit section 27 controls negative pressure means 28 on and off. In addition, negative pressure means 28 has a current change detecting section that detects change in the current.

Battery 29 supplies power to laser unit 26, electrical circuit section 27 and negative pressure means 28.

In blood test apparatus 21 having the above-described configuration (see FIG.4), when cover 22b opens, sensor outlet 24a opens and conveying means 30 inserts blood sensor 23 at the bottom in cartridge 24 between first holder 25a and second holder 25b. At this time, first holder 25a, blood sensor 23 and second holder 25b are closely attached to each other.

In addition, as shown in FIG.3, in blood test apparatus 21 when cover 22b is closed, gaps are defined between first holder 25a and the blood sensor 23 and between second holder 25b and blood sensor 23 by the operation of the pushing-up section as gap defining section 20.

Now, the operation of gap defining section 20 will be described in detail. FIG.8 is a cross sectional view of the puncturing section and its neighborhood for explaining the operation of the pushing-up section as the gap defining section.

FIG.8A is a cross sectional view showing the first state. In this first state, cover 22b is opened with respect to housing 22a and puncturing section 25 in housing body 22a is exposed outside. In this state, pressing projection 20b and supporting pawl 20c are apart from second holder 25b and blood sensor 23. Therefore, second holder 25b is biased toward first holder 25a by leaf spring 25c and is parallel to first holder 25a, and blood sensor 23 is sandwiched and fixed between these first holder 25a and second holder 25b. In this state, puncturing is performed.

FIG.8B is a cross sectional view showing the second state. In this second state, cover 22b rotates with respect to housing body 22a to cover housing body 22a.

That is, cover 22b is closed after puncturing is performed in the state shown in FIG.8A, so that the state shown in FIG.8B is defined. In this state, pressing projection 20b abuts on tongue piece 25p of second holder 25b from below and pushes up tongue piece 25p. Tongue piece 25p is pushed up by pressing projection 20b, so that second holder 25b rotates around spindle 25r as the supporting point, and therefore the leading edge of second holder 25b in the outlet 22f side moves toward pushing-up section body 20a.

By this means, the leading edge of second holder 25b inclines downward so as to be gradually spaced from first holder 25a, so that the area between first holder 25a and second holder 25b opens in the leading edge side of second holder 25b. Here, this area between the leading edge of second holder 25b and first holder 25a is in communication with outlet 22f.

As described above, second holder 25b rotates, so that first holder 25a is apart from first holder 25a. Specifically, second holder 25b moves in the direction to part from first holder 25a. At this time, since the punctured blood sensor 23 is placed on the top surface of the holder body, which sandwiches blood sensor 23, blood sensor 23 is moved with second holder 25b in the direction to part from first holder 25a. As a result of this, supporting pawls 20s project from the both sides of second holder 25b toward the upper part of the holder body of second holder 25b and abut on the both rims of the back surface of blood sensor 23. Then, while blood sensor 23 is supported by supporting pawls 20c, second holder 25b moves in the direction to part from first holder 25a.

By this means, blood sensor 23 is placed apart from first holder 25a and also placed apart from second holder 25b while being supported by supporting pawls 20c.

That is, in the state in which first holder 25a and second holder 25b are placed apart from one another, gap 20d is defined between blood sensor 23 and first holder 25a and also gap 20e is defined between blood sensor 23 and second holder 25b.

By this means, even if blood 10 applied to blood sensor 23 by puncturing, the location to which blood 10 is applied does not contact first holder 25a and second holder 25b, so that first holder 25a and second holder 25b are not stained with blood 10.

FIG.8C is a cross sectional view showing the third state. In this third state, sensor 23 is ejected between first holder 25a and second holder 25b which are open. While this ejecting operation is performed, cover 22b is closed and gap defining section 20 works, so that sensor 23 to which blood 10 is applied by puncturing can be ejected without staining first holder 25a and second holder 25b with blood 10. Here, in blood test apparatus 21 covered with cover 22b, the positions of the tips of supporting pawls 20c is lower than the position of the sandwiching surface of first holder 25a in height. Therefore, blood sensor 23 released from the sandwiched state slides on the tips of supporting paws 20c, moves by its own weight and slips down outside from outlet 22f while blood 10 is not in contact with blood test apparatus 21 itself.

As described above, in blood test apparatus 21, the operation of discarding blood sensor 23 after puncturing is as follows: upper and lower first holder 25a and second holder 25b facing to one another are placed apart from one another; and blood sensor 23 is supported by supporting pawls 20c projecting from beneath. By this means, the location of blood sensor 23, in which blood 10 is stored can be spaced from first holder 25a and second holder 25b, so that first holder 25a and second holder 25b are not stained with blood 10 applied to blood sensor 23.

In addition, as shown in FIG.8A, puncturing and measurement are performed in a state in which cover 22b is open, and pressing projection 20b as gap defining section 20 and supporting pawls 20c are placed apart from puncturing section 25 having first holder 25 and second holder 25b. By this means, pressing projection 20b as gap defining section 20 and supporting pawls 20c do not affect the positional relationship between first holder 25a, second holder 25b and sensor 23.

FIG.9 is a cross sectional view of blood sensor 23 stacked stored in cartridge 24.

In FIG.9, blood sensor 23 is composed of substrate 31, spacer 32 pasted on this substrate 31 and cover 33 pasted on this spacer 32, and has a rectangular plate-like shape.

Storing section 34 for storing blood is provided in blood sensor 23 in the position where laser light 26h passes through when sensor 23 is mounted in puncturing section 25.

Storing section 34 is a continuous hole composed of substrate hole 31a formed in substrate 31, spacer hole 32a formed in spacer 32 and cover hole 33a formed in cover 33.

Supply path 35 for blood 10 is a guide path to guide blood 10 stored in storing section 34 to detecting section 37 by capillary action, and its one end is coupled to storing section 34. In addition, the other end of this supply path 35 is coupled to air hole 38. The capacity of storing section 34 is about 1 µL, and the capacity of supply path 35 is about 0.15 µL. As described above, it is possible to perform blood test using a small amount of blood 10, so that the burden of the patient can be alleviated.

Positioning hole 36 penetrates blood sensor 23 and determines the mounting position of blood sensor. Positioning hole 36 penetrates blood sensor 23.

Detecting section 37 measures blood sugar level and so forth of blood 10.

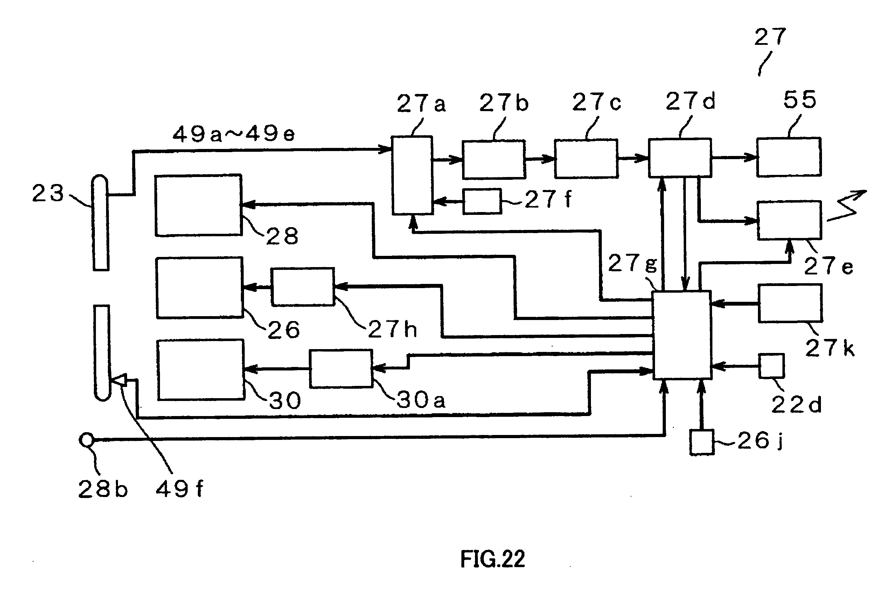

reagent 40 can be obtained by adding and dissolving PQQ-GDH (0.1 to 5.0 U/sensor), potassium ferricyanide (10 to 200 millimole), maltitol (1 to 50 millimole) and taurine (20 to 200 millimole) in a CMC solution of 0.01 to 2.0 wt% to prepare a reagent solution, by dropping the reagent solution on detection electrodes 171 and 173 (see FIG.22) formed on substrate 161 and drying. This reagent 40 is progressively degraded as a result of moisture absorbent.

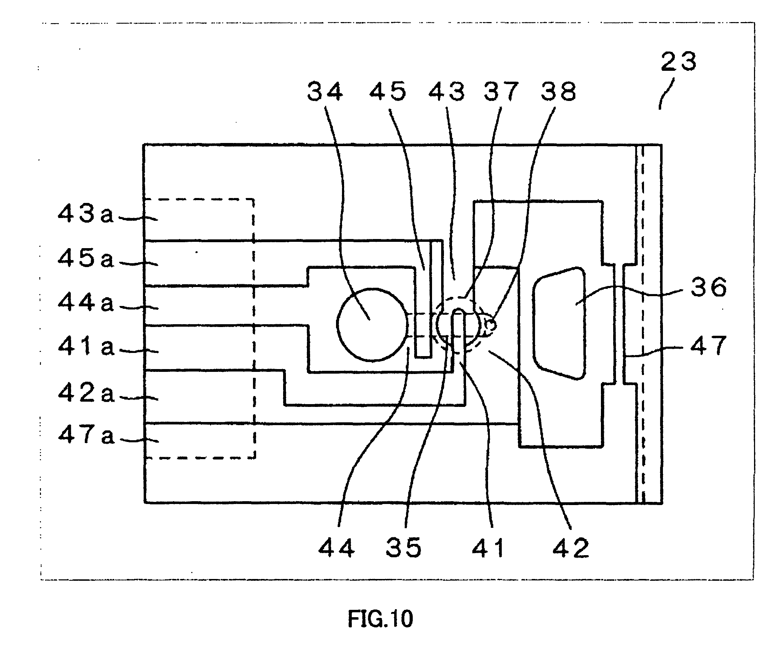

Here, an electrically conductive layer is formed on the top surface of the substrate 31 by the sputtering method or the vapor deposition method using materials such as gold, platinum, or palladium. Detection electrodes 41 to 45 (see FIG.10), connection electrodes 41a to 45a derived from these detection electrodes 41 to 45 and an identification electrode 47a are integrally formed by applying laser machining to the electrically conductive layer. Polyethylene terephthalate (PET) is used for the material for substrate 31, spacer 32 and cover 33. The material is used common between these components in this way, so that the management cost can be reduced.

FIG.10 is a perspective plane view of blood sensor 23 and FIG 11 is an external perspective view of blood sensor 23.

Blood sensor 23 shown in FIG.10 and FIG.11 has 6 electrodes. Storing section 34 is formed at approximately the center of plate-like shaped blood sensor 23, connection electrodes 41 a to 45a are formed at one end of blood sensor 23 and positioning section 36 is formed in the vicinity of the other end of blood sensor 23. Positioning section 36 is shaped as a hole, and has a trapezoidal shape narrowing toward storing section 34. Air hole 38 is formed between positioning section 36 and storing section 34.

Supply path 35 is provided toward detection electrode in storing section 34. One end of supply path 35 is connected to storing section 34. The other end of supply path 35 is coupled to air hole 38.

Storing section 34, detection electrode 44 connected to connection electrode 44a, detection electrode 45 connected to connection electrode 45a, again detection electrode 44 connected to connection electrode 44a, detection electrode 43 connected to connection electrode 43a, detection electrode 41 connected to connection electrode 41 a, again detection electrode 43 connected to connection electrode 43a and detection electrode 42 connected to connection electrode 42a, are provided on supply path 35, in the order described. In addition, reagent 40 (see FIG.9) is placed on detection electrodes 41 and 43. Identifying section 47 formed by a conductor pattern is formed between detection electrode 43 and identification electrode 47a.

Blood test apparatus 21 can identify whether sensor 23 is mounted in holding section 25, based on whether there is electrical conduction between connection electrode 43a and identification electrode 47a. That is, in case where there is not electrical conduction when conveying means 30 conveys blood sensor 23 to puncturing section 25, blood test apparatus 21 can display on display section 55 (see FIG.22) a warning indicating that sensor 23 is not mounted in holding section 25.

It is possible to store information of the calibration curve to be used and also manufacturing information by changing the electrical resistance of identifying section 47. Therefore, a blood test can be more accurately performed by using those information.

Here, although blood sensor 23 has 6 electrodes, the present embodiment is not limited to this, the electrodes may be configured by 5 electrodes and another configuration where there is no identification electrode 47, is also applicable. That is, it is possible to assign the identification electrode to one electrode which is, for example, a detection electrode other than a working electrode and a counter electrode (described later) that is not used to measure the components of blood, so that the electrodes can be configured by 5 electrodes.

In addition, although blood sensor 23 as shown in FIG.10 and FIG.11 has a rectangular plate-like shape, the shape of blood sensor 23 is not limited, and here, the shape of sensor 23 may be a square and a polygon other than a quadrangle.

Moreover, the shape of positioning section 36 is not limited, and here, positioning section 36 may be a hole shaped as a quadrangle, a polygon other than a quadrangle, a circle or an oval. Furthermore, positioning section 36 may not be a hole but may have a concave shape although not shown in the figure.

Next, the configuration of puncturing section 25 will be described in detail.

FIG.12 is a side view of first holder 25a constituting puncturing section 25, and FIG.13 is an external perspective view of first holder 25a, from the under surface 25e side.

Here, although first holder 25a has 6 electrodes as shown in FIG.13, the number of electrodes is not limited to this, the electrodes may be configured by 5 electrodes. In this case, the electrodes of blood sensor 23 sandwiched between first holder 25a and second holder 25b are configured by 5 electrodes.

As shown in FIG.12 and FIG.13, first holder 25a has sandwiching surface (bottom surface) 25e, which sandwiches blood sensor between first holder 25a and second holder 25b and inclined surface 25d that inclines upward from the end of under surface 25e in the side to which blood sensor 23 is conveyed. Inclined surface 25d and a part (tongue piece 25p) of second holder 25b form an opening in communication with the sandwiching area formed by first holder 25a and second holder 25b, and inclined surface 25d allows blood sensor 23 conveyed from the cartridge 24 side to slide and adequately guides blood sensor 23 to the sandwiching area.

In addition, hole 25g penetrating from top surface 25f to under surface 25e is formed in first holder 25a in the position where laser light as a perforatorium passes through. Here, when a needle puncturing device is employed instead of laser unit 26, a puncture needle is inserted through this hole 25g.

In addition, when blood sensor 23 is mounted in puncturing section 25, hole 25g corresponds to storing section 34 provided in blood sensor 23 (see FIG.9 to FIG.11). Moreover, this hole 25g is formed so as to bend in the lower part in a L-shape (when viewed laterally), by cutout section 25h provided by cutting out under surface 25e facing second holder 25b.

This tip of the L-shaped bending part corresponds to air hole 38 formed in blood senor 23. By this means, the effect of capillary action of the supply path formed in blood sensor 23 can be assured. In addition, a negative pressure produced by negative pressure means 28 is supplied through this hole 25g. Here, hole 25m (see FIG.14) in communication with this hole 25g is formed in second holder 25b.

Projection 25j is provided on under surface 25e, which is the sandwiching surface between inclined surface 25d and hole 25g. Projection 25j engages with positioning section 36 of blood sensor 23 facing under surface 25e and positions blood sensor 23 facing under surface 25e in the horizontal direction. Projection 25j has a trapezoidal shape narrowing toward hole 25g, viewed from under the surface 25e side. In addition, the thickness of projection 25j gradually increases from inclined surface 25d toward hole 25g. Specifically, projection 25j has one end in the inclined surface 25d and the other end in the hole 25g side. Among them, the one end in the inclined surface 25d is inclined such that the height of projection 25j gradually increases toward the other end in the hole 25g side. Therefore, when being inserted in puncturing section 25, blood sensor 23 slides along the gradient at the end of projection 25j in the inclined surface 25d side. When projection 25j positions in positioning hole 36, blood sensor 23 engages with the other end of projection 25j in the hole 25g side. By this means, blood sensor 23 inserted in puncturing section 25 is easily fixed to first holder 25a.

In addition, convex parts 25k is provided to project from first holder 25a, in the positions along both the rims of under surface 25e, right beside hole 25g. Convex parts 25k position blood sensor 23 in the direction orthogonal to the direction of insert of blood sensor 23 (i.e. width direction).

In addition, connectors 49 are provided at the end of under surface 25e, opposite to inclined surface 25d, that is at the end of under surface 25e in the direction of insert of blood sensor 23. Connectors 49 are formed corresponding to connection electrodes 41a to 45a and identifying electrode 47a of sandwiched blood sensor 23, and are connected to electrical circuit section 27 in contact with these connection electrodes 41a to 45a and identifying electrode 47a. Here, projection 25j and convex parts 25k may be provided in second holder 25b.

Moreover, guide cutout parts 251 opening downward, are formed on both side surfaces of first holder 25a. Guide pieces (including guide piece 25t) of second holder 25b are slidably arranged in those guide cutout parts 251, and guide the movement of second holder 25b when second holder 25b rotates with respect to first holder 25a.

FIG.14 is a sectional side view of second holder 25b constituting puncturing section 25.

As shown in FIG.14, the top surface of second holder 25b faces under surface 25e (see FIG.12 and FIG.13) of first holder 25a and has plate-like shaped holder body 25s that sandwiches blood sensor with under surface 25e of first holder 25a.

The surface of holder body 25s of second holder 25b, opposite to sandwiching surface 25v, that is, the under surface of the bottom in FIG.14 is puncturing positioning section 25w that position the skin to be punctured. Here, puncturing positioning section 25w configured by negative pressure chamber 28a, and enclosing section 25y that encloses the circumference of negative pressure chamber 28a and contacts the circumference of the punctured location of skin.

In second holder 25b, tongue piece 25p is formed, which inclines in the direction to part from sandwiching surface 25v of holder body 25s (downward), from the base end as the end of holder body 25s in the sensor outlet 24a side of sensor cartridge 24 (the end in the upstream of the direction in which sensor 23 is conveyed). The top surface (the surface on the sandwiching surface 25v side) of tongue piece 25p faces inclined surface 25d of first holder 25a, goes down toward second holder 25b in the base end side and forms an inclined surface having the same width as sandwiching surface 25v. By this means, the top surface (the surface in the sandwiching surface side) of tongue piece 25p forms the opening of puncturing section 25 in cooperation with inclined surface 25d of second holder 25b.

In addition, hole 25m in communication with hole 25g of first holder 25a is provided at the center of plate-like shaped holder body 25s of second holder 25b. Hole 25m is formed on holder body 25s in the position to communicate with hole 25g in the vertical direction when second holder 25b and first holder 25a are securely attached to one another and arranged parallel to one another.

Negative pressure chamber 28a coupled with hole 25m is formed so as to open downward in holder body 25s. Skin detecting sensor 28b is provided adjacent to this negative pressure chamber 28a. Here, skin detecting sensor 28b is provided on the edge surface of enclosing section 25y that determines the puncturing position with respect to skin by contact with skin. Skin detecting sensor 28b is connected to electrical circuit section 27 through connector 28e.

Here, a mechanical switch may be used as skin detecting sensor 28b, or one that detects electrical conduction may be used as skin detecting sensor 28b. In addition, skin detecting sensor 28b may be an optical sensor using light emitting diode and photo-transistor, or may be a magnetic sensor. With the present embodiment, an electrical sensor that detects electrical resistance of skin to be punctured is used as skin detecting sensor 28b.

In second holder 25b, the under surface of second holder 25b touches skin and defines a negative pressure in negative pressure chamber 28a, so that the skin swells in negative pressure chamber 28a. This swelling may be obtained by strongly pressing skin against second holder 25b. Here, although "negative pressure chamber 28a" is employed as a component name for convenience of explanation, negative pressure may not always be used.

Here, although second holder 25b has a configuration where spindle 25r is provided in the sensor outlet 24 side (see FIG.3 and FIG.4) and parallel to supporting point 22c (see FIG.3 and FIG.4), arrangement of spindle 25r is not limited to this on the condition that second holder 25b can rotate and move in the direction to contact first holder 25a and in the direction to part from first holder 25a. In other words, if first holder 25a and second holder 25a constituting puncturing section 25 face to one another and can move in the direction to contact one another and in the direction to part from one another, spindle 25r may be freely provided.

### (Alternative example)

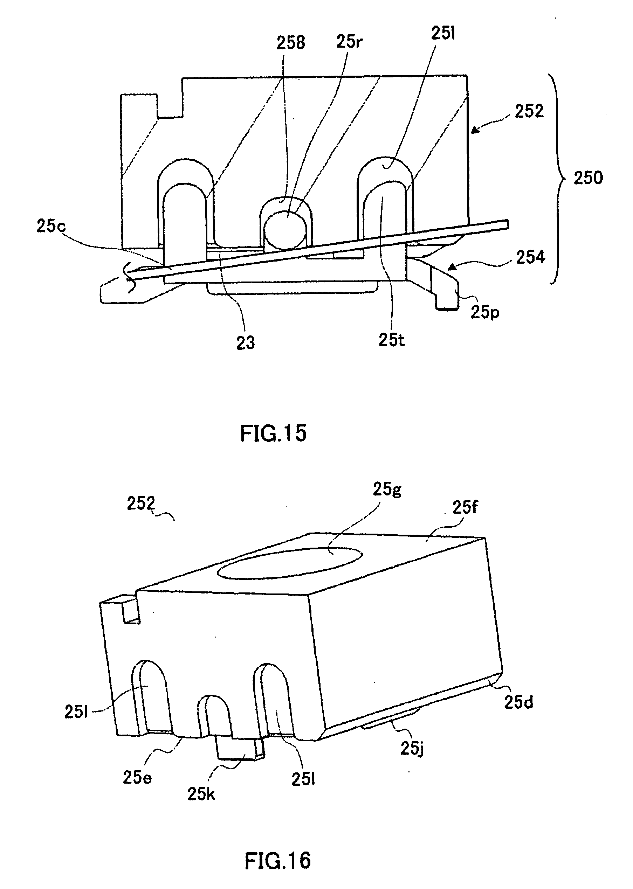

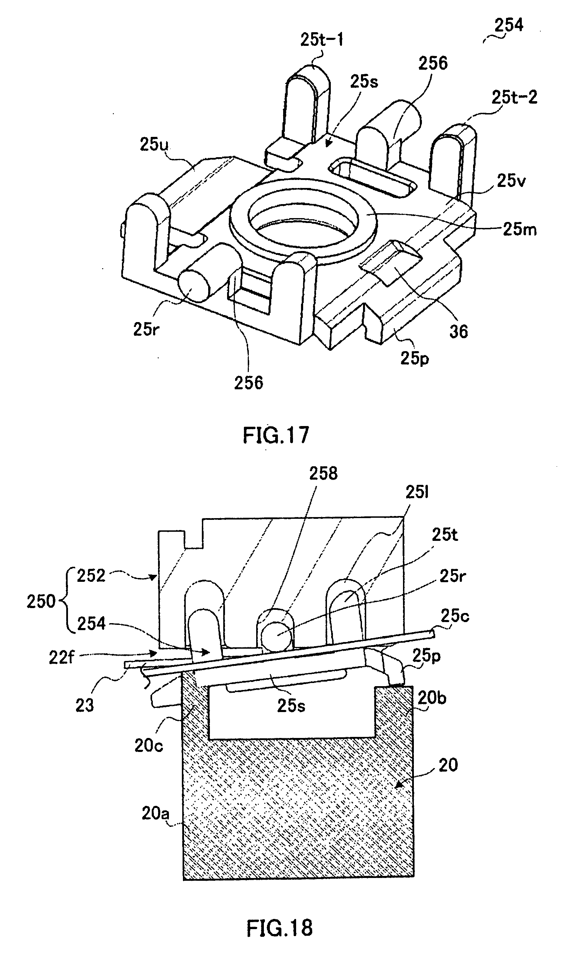

FIG.15 is a cross sectional view showing an alternative example of the puncturing section of the blood test apparatus according to embodiment 1. In addition, FIG.16 is a perspective view of first holder 252 constituting an alternative example of the puncturing section of the blood test apparatus according to embodiment 1, and FIG.17 is a drawing showing second holder 254 constituting an alternative example of the puncturing section and is perspective view of the second holder from the top surface. Moreover, FIG.18 is a cross sectional view explaining operation of the gap defining section for an alternative example of the puncturing section of the blood test apparatus. Here, in the alternative example of blood test apparatus 21 according to embodiment 1 shown in FIG.15 to FIG.18, the configurations of first holder 25a and second holder 25b constituting the puncturing section of the blood test apparatus are different from those of the above-described first holder 25a (see FIG.3 to FIG.8) and the above-described second holder 25b (see FIG.3 to FIG.8), but the other configurations are the same. Therefore, only the different points will be described, and the same components will be assigned the same reference numerals and the same names and the explanation will be omitted.

In puncturing section 250, which is an alternative example of the above-described puncturing section of the blood test apparatus according to embodiment 1, spindle 25r of second holder 254 movably provided in the direction to contact first holder 252 and in the direction to part from first holder 252 is located near the center of top surface 25v. That is, puncturing section 250 has the same configuration as the above-described puncturing section 25, except for the positioning of spindle 25r.

Specifically, first holder 252 as shown in FIG.16 has a substantially rectangular solid shape and has basically the same configuration as the above-described first holder 25a except spindle guide grooves 258 are formed on its both sides, in addition to guide cutout sections 251.

That is, first holder 252 has sandwiching surface (under surface) 25e to sandwich blood sensor 23 between first holder 252 and second holder 254 and inclined surface 25d to incline upward from the end of under surface 25e in the side to which blood sensor 23 is conveyed. Inclined surface 25d and a part (tongue piece 25p) of second holder 25b form an opening in communication with a sandwiching area formed with second holder 25b, and inclined surface 25d allows blood sensor 23 conveyed from the cartridge 24 side to slide and adequately guides blood sensor 23 to the sandwiching area. Here, hole 25g penetrating from top surface 25f to under surface 25e is formed in first holder 252, and laser light as a perforatorium passes through this hole 25g. When blood sensor 23 is mounted in puncturing section 250, this hole 25g corresponds to storing section 34 provided in blood sensor 23 (see FIG.9 to FIG. 11). Here, when a needle puncturing device is employed instead of laser unit 26, a puncture needle passes though this hole 25g.

Projection 25j that engages with positioning hole 36 (see FIG.17) of blood sensor 23 to position blood sensor 23 located to face under surface 25e in the horizontal direction and convex parts 25k that position blood sensor in the direction orthogonal to the inserting direction of blood sensor 23 (i.e. width direction of blood sensor 23) project on under surface 25, which is the sandwiching surface.

Guide cutout parts 251 opening downward are formed on both side surfaces of first holder 252. On each side surface, one guide cutout part 251 is formed in the vicinity of the base end, which is the end of first holder 252 in the sensor outlet 24a side (the inserting inlet of puncturing section 250 side) where blood sensor 23 is conveyed outside from sensor cartridge 24, and the other guide cutout part 251 is formed in the vicinity of the leading end, which is the end of first holder 252 in the outlet side that ejects blood sensor 23 outside from puncturing section 250. Guide pieces 25t and 25t-1 of second holder 254 (see FIG.17) are slidably disposed in those guide cutout parts 251, so that the guide cutout parts 251 guide the movement of second holder 254 when second holder 254 rotates with respect to first holder 252.

Supporting guide groove 258 is formed to open downward at the center of each side surface of first holder 252.

Spindle guide pieces 256 (see FIG.17) for second holder 254, which project from the second holder 254 side are slidably inserted in supporting guide grooves 258.

As shown in FIG.17, supporting guide pieces 256 are formed so as to rise from approximately the centers of both rims of holder body 25s of second holder 254 along the inserting direction of blood sensor 23, and spindle 25r projects outward orthogonal to the direction in which supporting guide pieces 256 rise. Spindle 25r is parallel to supporting point 22c (see FIG.3 and FIG.4) and are disposed in supporting guide grooves 258 of first holder 252 so as to be able to move in the direction to contact first holder 252 and in the direction to part from first holder 252 (i.e. vertical direction).

Second holder 254 as shown in FIG.17 has the same configuration as the above-described second holder 25b (see FIG.3 to FIG.8 and FIG.14) except for positioning of spindle 25r. That is, second holder 254 has plate-like shaped holder body 25s and has the top surface facing under surface 25e of first holder 252. Blood sensor 23 is sandwiched between this top surface of holder body 25s and under surface 25e of first holder 252. In addition, holder body 25s of second holder 254 is provided with a puncturing positioning section (not shown) that positions the skin to be punctured on the surface opposite to sandwiching surface 25v, as with second holder 254. Moreover, second holder 254 has tongue piece 25p, hole 25m and so forth.

In puncturing section 250 has first holder 252 and second holder 254 configured as described above, supporting guide pieces 256 having spindle 25r at their tips are disposed in supporting guide grooves 258 of first holder 252 so as to be able to move in the vertical direction. By this means, when rotating with respect to first holder 252, second holder 254 is allowed to move in the direction to contact first holder 252 and in the direction to part from first holder 252 but limited to move in the direction in which first holder 252 slides. Here, first holder 252 and second holder 254 are mounted in the housing body as with the above-described first holder 25a and second holder 25b.

In puncturing section 250 as shown in FIG.15, second holder 254 is pressed toward first holder 252 by leaf spring 25c provided in housing body 22a (see FIG.3) through spindle 25r located at approximately the center of top surface 25v, which is the sandwich surface and is kept parallel to first holder 252, in the state in which inserted blood sensor 23 is sandwiched and fixed between first holder 252 and second holder 254.

As described above, second holder 254 is supported at approximately its center part through spindle 25r with respect to first holder 252. Therefore, since the finger touches the vicinity of spindle 25r when puncturing is performed while the finger touches the bottom surface of second holder 254, puncturing can be performed in stable condition.

When cover 22b (see FIG.3 and FIG.4) is closed after puncturing is performed, the state as shown in FIG.18 is made. In this state, pressing projection 20b abuts on tongue piece 25p of second holder 254 from beneath, so that tongue piece 25p is pushed up by this pressing projection 20b. Tongue piece 25p is pushed up by pressing projection 20b, so that second holder 254 rotates around spindle 25r and the leading edge of second holder 254 in the outlet 22f side moves to the pressing section body 20a side.

Second holder 254 rotates around spindle 25r, so that second holder 254 opens at its leading edge in the outlet 22f side. As described above, the opening is defined between the leading edge of second holder 254 and first holder 252, so that outlet 22f is located in the position on the extended line of the direction in which blood sensor 23 is ejected.

As described above, second holder 254 rotates to move in the direction in which the leading edge of second holder 254 in the outlet 22f side is placed apart from first holder 252. As a result of this, supporting pawls 20c project from both sides of second holder above holder body 25s. Edges of supporting pawls 20c abut on both rims Al (see FIG. 11) of the bottom surface of blood sensor 23 placed on second holder 254 to part blood sensor 23 from second holder 254. By this means, while being placed apart from first holder 252 and also placed apart from second holder 254, blood sensor 23 is supported by supporting pawls 20c.

That is, in the state in which first holder 252 and second holder 254 are placed apart from one another, blood sensor 23 placed between first holder 252 and second holder 254 has a gap between first holder 252 and itself and a gap between second holder 254 and itself. By this means, even if blood 10 is applied to blood sensor 23 by puncturing, the location to which blood is applied does not touch first holder 252 and second holder 254, so that first holder 252 and second holder 254 can be kept sanitary after puncturing is performed.

Next, the inner configuration of cartridge 24 will be described in detail. FIG.19 is a perspective plane view of cartridge 24.

In cartridge 24 shown in FIG.19, case 24b is made of resin and so forth and has a rectangular solid shape, and positioning concave part 24t is provided on one side surface of case 24b. When cartridge 24 is inserted in housing body 22a of housing 22, a positioning convex part configured by a leaf spring and so forth provided in housing body 22a fits in this positioning concave part 24t. By this means, cartridge 24 is positioned in housing body 22a.

sensor chamber 24c and drying chamber 24d are arranged side by side in case 24b. Sensor chamber 24c and drying chamber 24d communicate through passage 24e in case 24b. Blood sensors 23 are stacked and stored in sensor chamber 24c. In addition, desiccant 50 is stored in drying chamber 24d.

Negative pressure path 24f is formed so as to lead to outside, on top surface 24 of case 24b located in the upper part of sensor chamber 24c. This negative pressure path 24f has a cylindrical shape and is coupled to negative pressure means 28 (see FIG.3 and FIG.4) inside housing body 22a. A negative pressure is supplied from negative pressure means 28 to sensor chamber 24c through this negative pressure path 24f, so that dampness in sensor chamber 24c is reduced. Spring 24j biases stacked and stored blood sensors 23 downward through pressing plate 24g. Hole 24h corresponding to storing section 34 of stored blood sensor 23 is provided at approximately the center of pressing plate 24g and communicates storing section 34 of blood sensor 23.

Therefore, a negative pressure introduced from negative pressure path 24f reduces dampness in storing section 34 of each blood sensor 23 through this hole 24h and prevents the performance of reagent 40 (see FIG.2) provided in each sensor 23 from deteriorating due to dampness. In addition, desiccant 50 is stored in drying chamber 24d, and drying air supplied from drying chamber 24d dries storing section 34 of each sensor 23 through passage 24e as with desiccant 50, so that reagent 40 is protected from deteriorating due to dampness.

That is, since cartridge 24 has drying chamber 24d in case 24b, the housing capacity of drying gas can be increased, so that sensor 23 can be prevented from deteriorating for a long time. Since a negative pressure is supplied in case 24b through negative pressure path 24f in the present embodiment, the size of desiccant 50 can be reduced.

Slider plate 24k that slides in the direction intersecting the direction in which blood sensors 23 are stacked, and plate drive section 30b that moves slider plate 24k, are located below sensor chamber 24c. Slider plate 24k and plate drive section 30b constitute conveying means 30.

A step surface on which one blood sensor 23 is placed is formed on a part of the top surface of slider plate 24k. This step surface moves from the bottom of sensor chamber 24c to the outside of sensor cartridge 24 through sensor outlet 24a by sliding slider plate 24k.

That is, when blood sensor 23 at the bottom among blood sensors 23 stacked and stored in cartridge 24 is placed on the step surface, this blood sensor 23 is separated from stacked blood sensors 23 on the step part by the sliding of slider plate 24 and is pushed out to the sensor outlet 24a side. By this means, blood sensor 23 at the bottom among blood sensors stacked is conveyed outside from senor outlet 24a by slider plate 24k.

Slider plate 24k and plate drive section 30b may be a conveying belt suspending between pulleys, or may be provided by forming a spiral groove on a cylindrical body. Anyway, this plate drive section 30b is driven by drive section 30a (see FIG.3 and FIG.4). Here, conveying means 30 may be configured by slider plate 24k and plate drive section 30b, and also drive section 30a.

In cartridge 24, shatter 24n that opens and closes sensor outlet 24a is formed near the lower part of one side surface 24s (left side wall in FIG.19) adjacent to top surface 24p.

Shutter 24n is disposed between one side surface 24s and rib 24r apart from one side surface 24s at a predetermined distance and projecting outward, and pin 24z of shatter 24n is slidably inserted in a long hole (not shown) of rib 24r. This sliding allows shutter 24n to move up and down on the surface of one side surface 24s, at the bottom end part of one side surface 24s. When shatter is located at the top end, sensor outlet 24a closes, and, when shatter 24n is located at the bottom end, sensor outlet 24a opens.

Here, shatter 24n opens and closes in conjunction with the opening and closing of cover 22b (see FIG,3 and FIG.4). That is, when cover 22b opens, a state in which sensor outlet 24a is exposed outside is made, that is, shatter 24 is in "open" state. At this time, a configuration may be applicable in which shatter 24n moves downward by its own weight, or a configuration may be applicable such that shatter 24n is moved by a bias member for biasing downward. In addition, closing of cover 22b makes a state in which shatter 24n moves upward by a pushing up member provided in the cover 22b side to cover sensor outlet 24a, that is, shatter 24 is in "closed" state. When shatter 24n is in "closed" state, the inside of case 24b is made airtight, so that reagent 40 in sensor 23 is protected from dampness. For example, pressing projection 20b (see FIG.18, etc.) may be used as a pressing member for moving shatter 24n upward when cover 22b is closed.

In addition, sensor outlet 24a is closed by shatter 24n, so that the inside of sensor chamber 24c is made airtight and a negative pressure supplied from negative pressure path 24f is held in cartridge 24n.

FIG.20 is a cross sectional view of laser unit 26 as a puncturing means.

In FIG.20, laser unit 26 is composed of oscillating tube 26a and cylindrical body 26b coupled to this oscillating tube 26a. Er:YAG (yttrium, aluminium, garnet) laser crystal 26c and flash light source 26d are housed in oscillating tube 26a.

Flash light source 26d is connected to high voltage generating circuit 27h provided in electrical circuit section 27. This high voltage generating circuit 27h is provided with charge completion detecting section 27m (described later) that detects the completion of charging.

Partially transmitting mirror 26e having a transmissivity of about 1 % is mounted on one end of oscillating tube 26a, and total reflecting mirror 26f is mounted on the other end of oscillating tube 26a. Convex lens 26g is mounted in cylindrical body 26b before partially transmitting mirror 26e and set to focus laser light 26h on a position below the surface of the skin of the patient.

Laser unit 26 having the configuration described above will be explained in detail. Here, skin detection sensor 28b (see FIG.1) detects skin 9 and in a state in which this detection signal has been already inputted, flash light source 26d excites by pressing puncturing button 26j.

Light emitted from this flash light source 26d enters Er:YAG laser crystal 26c. The light is then continuously reflected between total reflecting mirror 26h, YAG laser crystal 26c and partially transmitting mirror 26e, resonates, and is amplified. Part of amplified laser light passes through partially transmitting mirror 26e by induced emission. This laser light 26h passing through partially transmitting mirror 26e is emitted through focusing lens 26g and focuses under skin 9. The appropriate focal depth for puncturing is 0.1 mm to 1.5 mm from the surface of skin 9. In the present embodiment, the puncturing depth is 0.5 mm.

The present embodiment employs laser unit 26 that can puncture skin 9 of the patient in a noncontact state, so that sanitation is assured. In addition, laser unit 26 has no moving components, so that there is little malfunction. Here, this laser light 26h punctures skin with the voltage about 300V. Therefore, the patient feels little pain.

FIG.21 is a cross sectional view of the puncturing section when puncturing-measurement operation is performed by the blood test apparatus and its nearby primary parts.

As shown in FIG.21, when puncturing-measurement operation is performed, negative pressure chamber 28a on the under surface of second holder 25b is connected to negative pressure means 28 (see FIG.3 and FIG.4) through hole 25m formed in second holder 25b and hole 25g of first holder 25a. Skin detecting sensor 28b is provided in the surrounding part forming the surround of negative chamber 28a. Signals from connection electrodes 41a to 45a and identifying electrode 47a (see FIG.10) of blood sensor 23, sandwiched between fist holder 25a and second holder 25b, are guided to electrical circuit section 27 through connectors 49.

Skin 9 touches second holder 25b of blood test apparatus 21 having the configuration described above and is detected by skin detecting sensor 28b, and then puncturing button is pressed, so that laser unit 26 emits laser light 26h. Here, another configuration is applicable where the output signal from skin detecting sensor 28b is outputted to display section 55 (see FIG.22) to allow the user to visually recognize the contact state.. In this case, the signal from skin detecting sensor 28b may not be inputted to laser unit 26.

Laser light 26h passes straight through hole 25g of first holder 25a, storing section 34 of blood sensor 23 and hole 25m of second holder 25b to puncture skin 9. When skin 9 is punctured, blood 10 exudes from skin 9 and forms blood droplet 10a.

This blood droplet 10a is taken into detecting section 37 (see FIG.10) and reacts with reagent 40. The signal resulting from the reaction of reagent 40 and blood droplet 10a is measured by electrical circuit section 27 through connectors 49.

FIG.22 is a block diagram of electrical circuit section 27 and its neighborhood. In FIG.22, connection electrodes 41a to 45a and identifying electrode 47a (see FIG.10) are connected to switching circuit 27a through connectors 49a to 49f. The output of this switching circuit 27a is connected to the input of current/voltage convertor 27b. Then, the output of this current/voltage convertor 27b is connected to the input of computing section 27d through analog/digital convertor (hereinafter referred to as "A/D convertor") 27c. The output of this computing section 27d is connected to display section 55 made of liquid crystal and transmitting section 27e. In addition, reference voltage source 27f is connected to switching circuit 27a. Here, this reference voltage source 27f may be a ground potential.

Control section 27g controls the entire operation of the test apparatus according to the present invention. The output of this control section 27g is connected to high voltage generating circuit 27h connected to laser unit 26, a control terminal of switching circuit 27a, computing section 27d, transmitting section 27e, negative pressure means 28 and drive section 30a coupled to conveying means 30.

In addition, opening and closing sensor 22d that detects opening and closing of cover 22b, puncturing button 26j, detecting sensor 28b, computing section 27d, timer 27k and connector 49f connected to identification electrode 47a are connected to the input of control section 27g.

Next, operation of electrical circuit section 27 will be described. First, puncturing button 26j is pressed to puncture skin 9 by laser unit 26. Here, when skin detecting sensor 28b detects skin 9, negative pressure means 28 may be activated to define a negative pressure in negative pressure chamber 28a. In addition, laser unit 26 may emit laser light 26h (see FIG.21), provided that skin detecting sensor 28b inputs the skin detecting signal.

Then, the property of blood 10 exuding by puncturing is measured. In measurement operation, switching circuit 27a is switched, and detection electrode 41 (see FIG.10) is connected to current/voltage convertor 27b. In addition, detection electrode 42 to be a detecting electrode for detecting an inflow of blood 10 is connected to reference voltage source 27f. Then, a constant voltage is applied between detection electrode 41 and detection electrode 42. In this state, a current flows between detection electrodes 41 and 42 if blood 10 flows in. This current is converted into a voltage by current/voltage convertor 27b, and the voltage value is converted into a digital value by A/D convertor 27c. Then, the digital value is outputted to computing section 27d. Computing section 27d detects that blood 10 has sufficiently flown in, based on the digital value, and outputs this fact to the control section 27g.

Here, at this time, the operation of negative pressure means 28 is turned off by a command from control section 27g.

Next, glucose being a component of blood will be measured. To measure the glucose level, first, switching circuit 27a is switched by a command from control section 27g, and detection electrode 41 to be a working electrode for measuring the glucose level is connected to current/voltage convertor 27b. In addition, detection electrode 43 to be a counter electrode for measuring the glucose level is connected to reference voltage source 27f.

Here, for example, while the glucose in blood and its oxidation-reduction enzyme react for a given period of time, current/voltage convertor 27b and reference voltage source 27f are turned off. Then, after the certain period of time (1 to 10 seconds) passes, a certain voltage (0.2 to 0.5 V) is applied between detection electrodes 41 and 43 by a command from control section 27g. By this means, a current flows between detection electrodes 41 and 43. This current is converted into a voltage by current/voltage convertor 27b, and the voltage value is converted into a digital value by A/D convertor 27c and outputted to computing section 27d. Computing section 27d converts this digital value to a glucose level.

Next, after the glucose level is measured, a Hct value will be measured. The Hct value will be measured as follows. Firstly, switching circuit 27a is switched by a command from control section 27g. Then, detection electrode 45 to be a working electrode for measuring the Hct value is connected to current/voltage convertor 27b. In addition, detection electrode 41 to be a counter electrode for measuring the Hct value is connected to reference voltage source 27f.

Next, a certain voltage (2V to 3V) is applied between detection electrodes 45 and 41 from current/voltage converter 27b and reference voltage source 27f, by a command from control section 27g. The current that flows between detection electrodes 45 and 41 is converted into a voltage by current/voltage convertor 27b, and the voltage value is converted into a digital value by A/D converter 27c and is outputted to computing section 27d. Computing section 27d converts this digital value to the Hct value.

By using those Hct value and glucose level resulting from measurement and referring to a calibration curve or calibration curve table determined in advance, the glucose level is corrected by the Hct value and the correction result is displayed on display section 55. This calibration curve or calibration curve table is determined by identifying section 47 in blood sensor 23. In addition, the correction result by the calibration curve or calibration curve table is transmitted from transmitting section 27e to an injection device for injecting insulin. Although a radio wave may be used for this transmission, transmission is preferably performed by optical communication that does not interfere with medical equipment.

When the dose of insulin to administer is automatically set by transmitting corrected measurement data from transmitting section 27e in this way, setting the dose of insulin to be administered by the patient is not required, which eliminates botheration with setting. Moreover, since the dose of insulin can be set in the injection device without human work, setting error can be prevented.

Although an example of glucose measurement has been described above, the blood test apparatus is applicable to measurement of blood components other than glucose such as lactate acid or cholesterol levels by changing reagent 40 of sensor 23.

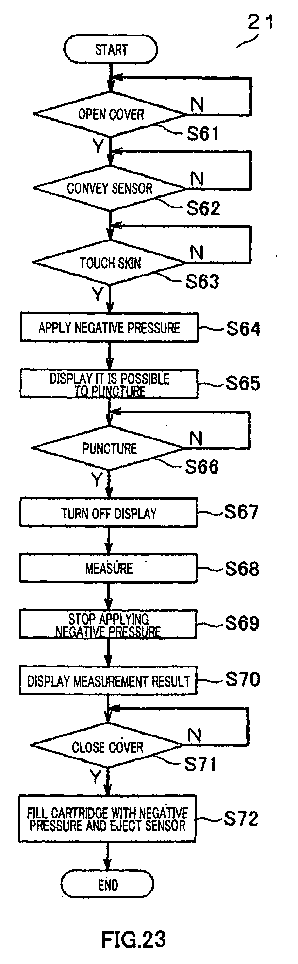

Next, a test method using blood test apparatus 21 will be described with reference to the flowchart of FIG.23. First, in step S61, the user opens cover 22b of blood test apparatus 21. When cover 22b is opened, shutter 24n of sensor outlet 24a provided in cartridge 24 opens in conjunction with the opening of cover 22b.

When cover 22b is opened (step S61: YES), the step moves to step S62, and in step S62, slider plate 24k constituting the conveying means is moved toward sensor outlet 24a. Here, plate moving section 30 operates by driving drive section 30a to slide slider plate 24k, so that only one blood sensor 23 at the bottom is separated, among blood sensors 23 stacked and stored and is conveyed from sensor outlet 24a to puncturing section 25. Conveyed blood sensor 23 is sandwiched between first holder 25a and second holder 25b of puncturing section 25. That is, a state in which blood sensor 23 is mounted in puncturing section 25 is defined.

Whether blood sensor 23 has been conveyed can be performed by detecting electrical conduction of connection electrode 43a and identifying electrode 47a of blood sensor 23. After that, slider plate 24k returns to the standby state by the operation of plate moving section 30. By this means, it is possible to convey the subsequent blood sensor 23. Control section 27g displays an indication to prompt puncturing section 25 to contact skin 9 on the display section 55 (see FIG.22), based on detection of electric conduction of connection electrode 43a and identifying section 47a. Following the display, the step moves to step S63.

After blood sensor is conveyed, in step S63, skin detecting sensor 28b detects whether the user touches blood test apparatus 21 with his/her skin 9 according to the command of display section 55. If blood test apparatus 21 is not in contact with skin 9 (step S63: NO), control section 27g waits until skin 9 touches blood test apparatus 21. If skin detecting sensor 28b detects contact with skin 9 in step S63 (step S63: YES), the step moves to step S64.

When skin 9 touches blood test apparatus 21, control section 27g operates negative pressure means 28 to define a negative pressure in negative pressure chamber 28a provided in holding section 25 in step S64. In addition, control section 27g operates high voltage generating circuit 27h to start charging. Here, 4 to 5 seconds is enough time period to apply a negative pressure. By applying a negative pressure, skin 9 swells as shown in FIG.21.

When the current is changed by the operation of negative pressure means 28 and charging with high voltage is completed (1 to 10 seconds) by high voltage generating circuit 27h, or when timer 27K shows that a predetermined time has passed, blood test apparatus 21 judges that the surface of skin 9 in storing section 34 sufficiently swells by a negative pressure and charging required for puncturing has been completed, and the step moves to step S65. Here, if change in the current by operation of negative pressure means 28 is not detected, or if timer 27k does not measure the passage of a predetermined time period, control section 27g judges that preparation for puncturing is not completed and waits until preparation for puncturing is completed.

In step S65, control section 27g displays that "it is possible to puncture the skin" on display 55 and the step moves to step S66.

In step S66, control section 27g waits press of puncturing button 26j, and when puncturing button 26j is pressed, blood test apparatus 21 (control section 27g) punctures skin 9. Here, instead of pressing puncturing button 26j, puncturing may be performed automatically by blood test apparatus 21, if the following conditions are all satisfied: the predetermined time period has been passed; change in the current by operation of negative pressure means 28 is detected; the skin detecting sensor checks that skin 9 contacts the blood test apparatus; and so forth.

After puncturing is performed in step S66, the step moves to step S67. In step S67, blood test apparatus 21 (control section 27g) turns off once the display in step S65, and the step moves step S68.

In step S68, blood test apparatus 21 (control section 27g) measures blood sugar level and so forth of blood 10 (see FIG.21) exuding by puncturing skin 10(9) using detecting section 37. Here, the time required for the measurement is about 3 to 5 seconds. In measurement of step S68, first, blood 10 exuding by puncturing skin 9 is taken into storing section 34 of blood sensor 23 (see FIG. 9 and FIG.10), and then blood 10 taken into this storing section 34 is introduced into detecting section 37 at a breath (at a constant flow rate) by capillary action of supply path 35. By this means, the blood sugar level of blood 10 is measured.

Next, in step S69, blood test apparatus 21 (control section 27g) stops negative pressure means 28 and the step moves step S70.

In step S70, blood test apparatus 21 (control section 27g) displays the measured blood sugar level and so forth on display section 55, and the step moves to step S71. Here, the measurement result of blood sugar level in step S70 may be automatically transmitted from transmitting section 27e to another equipment such as an injection device. Now, the measurement of blood 10 is completed. Here, blood test apparatus 21 may turn off negative pressure means 28 at the time blood sensor 10 reaches detection electrode 42.

In step S71, the user closes cover 22b of blood test apparatus 21. In conjunction with operation to close cover 22b, shatter 24n of cartridge 24 closes, so that sensor outlet 24a is closed. In addition, in step S71, opening and closing sensor 22d detects whether cover 22b of blood test apparatus 21 is closed. Here, if cover 21b of blood test apparatus 21 is not closed, control section 27b waits in this state. When cover 22b is closed, opening and closing sensor 22d detects closing of cover 22b and reports that to control section 27g.

In step S72, gap defining section 20 operates by closing cover 22b, so that gaps are defined between first holder 25a and blood sensor 23 and between the second holder 25b and blood sensor 23. Therefore, those gaps allow blood sensor 23 to which blood 10 is applied to eject from outlet 22f without staining first holder 25a and second holder 25b with blood 10.

In addition, in step S72, receiving a closing signal from cover detecting sensor 22e, control section 27g supplies and fills cartridge 24 with a negative pressure for a predetermined time period.. By this means, deterioration of blood sensor 23 due to dampness is delayed. In step S72, control section 27g stops supplying a negative pressure to cartridge 24 at the time filling cartridge 24 with a negative pressure is completed.

As described above, in blood test apparatus 21 according to the present embodiment, when cover 22b covers housing body 22a after puncturing-measurement, first holder 25a and second holder 25b are placed apart from the location of blood sensor 23, to which blood 10 is applied.

By this means, blood sensor 23 to which blood 10 is applied can be ejected in a state in which both first holder 25a and second holder 25b are placed apart from blood sensor 23. That is, blood senor 23 can be ejected without staining first holder 25a, second holder 25b and also housing 22 with blood 10 applied to itself, so that sanitation is assured.

In addition, sensor outlet 24a of cartridge 24 is opened in conjunction with opening of cover 22b, and sensor outlet 24a is closed only by closing cover 22b. Therefore, any extra operation is not required in order to only open and close sensor outlet 24a, so that the burden is eliminated.

Moreover, puncturing is performed after blood sensor 23 is conveyed unlike conventional embodiments. Since this puncturing is performed through storing section 34 (see FIG.9, FIG.10 and FIG.21), all blood 10 exuding by puncturing is stored in storing section 34. By this means, blood 10 is efficiently used to measure the blood sugar level reliably, and the burden on the patient can be minimized.

In the blood test apparatus as described above, puncturing section 25 is configured by first holder 25a and second holder 25b that sandwich blood sensor 23, and has the gap defining section that defines the gaps between blood sensor 23 and first holder 25a and between blood sensor 23 and second holder 25b when first holder 25a and second holder 25b are placed apart from one another. When first holder 25a and second holder 25b are placed apart from one another, the gaps are defined between blood sensor 23 and first holder 25a and between blood sensor 23 and second holder 25b, so that puncturing section 25 is not stained with blood 10 applied to blood sensor 23. Therefore, the puncturing section is kept clean. That is, after blood is measured using blood sensor 23 held by the holding section such as the puncturing section, used blood sensor 23 can be discarded without staining the holding section with blood, so that the puncturing section can be kept clean after measurement.

Here, control section 27g may issue a warning to the patient by means of buzzer or blink of LED after a predetermined time period passes since the opened state of cover 22b of housing 22 is detected, using the detection signal of opening and closing of cover 22b by opening and closing sensor 22d.

### (Embodiment 2)

Although embodiment 1 describes the gap defining section as the pushing-up section mainly provided in cover 22b, the present embodiment describes the blood test apparatus having the gap defining section that is provided in the puncturing section. Here, this blood test apparatus of embodiment 2 has the same configuration as blood test apparatus 21 of embodiment 1 shown in FIG.3, except for the configuration of the gap defining section and the first holder and the second holder constituting puncturing section 25. Therefore, the same components as in embodiment 1 will be assigned the same reference numerals and detailed description will be omitted.

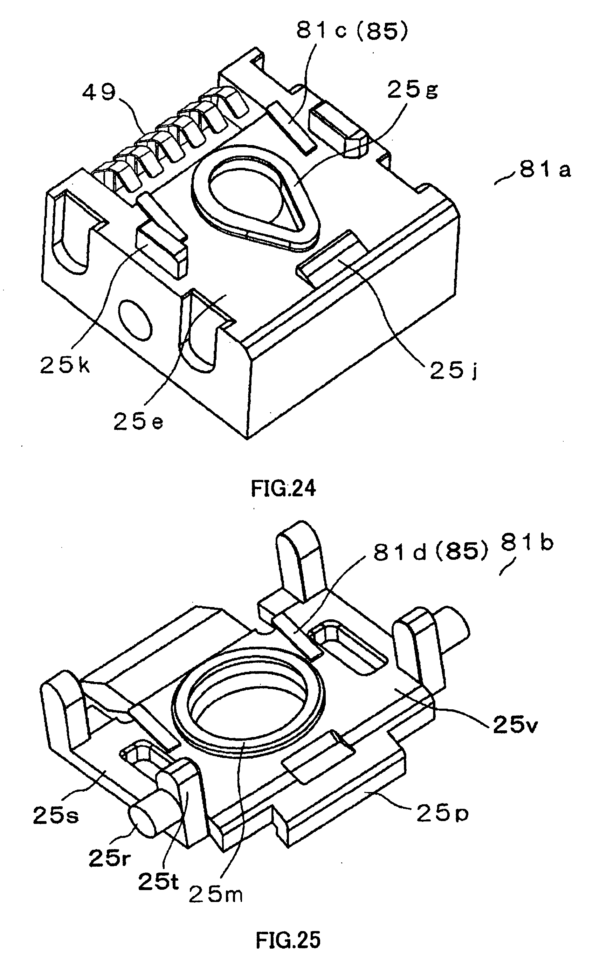

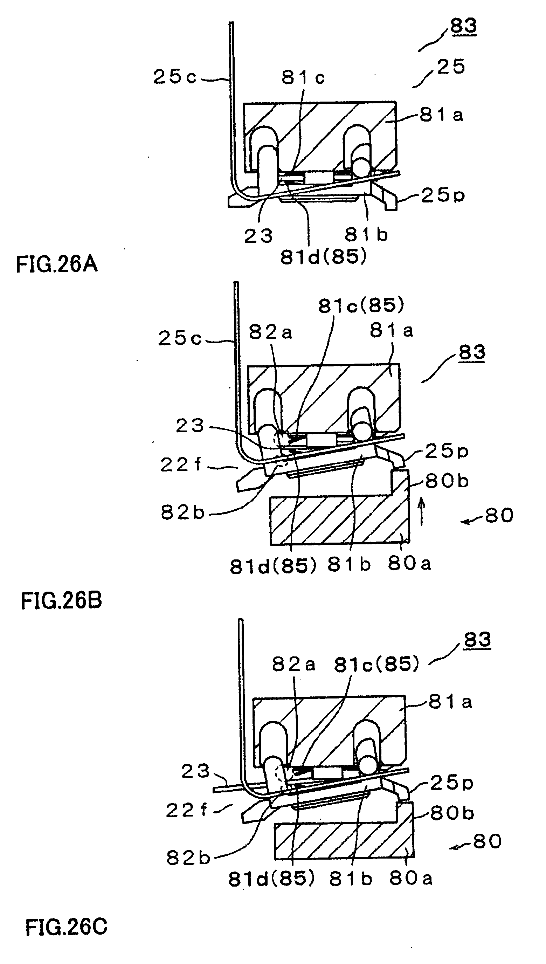

FIG.24 is a perspective view of the first holder constituting puncturing section in the blood test apparatus according to embodiment 2 from the bottom, and FIG.25 shows second holder constituting the above-mentioned puncturing section from the top. In addition, FIG.26 is a cross sectional view explaining operation of gap defining section 80.

Blood test apparatus 83 (see FIG.26) of embodiment 2 has the same configuration as blood test apparatus 21 according to embodiment 1 except first holder 81a as shown in FIG.24 and second holder 81b as shown in FIG.25 constitute puncturing section 81 (see FIG.2). In addition, blood test apparatus 83 (see FIG.26) includes pushing-up section 80 (see FIG.26B and FIG.26C) instead of pushing-up section 20 provided in cover 22b of blood test apparatus 21 according to embodiment 1 described above.

In embodiment 2, gap defining section 85 is composed of plate-like elastic bodies (see FIG.24 to FIG.26) of first holder 81a and second holder 81b and pressing projection 80b.

As shown in FIG.24, first holder 81a has approximately the same configuration as first holder 25a of embodiment 1 and includes, on its under surface 25e, plate-like elastic bodies 81c (resin leaf springs), which are elastic body projecting from under surface 25e. Here, under surface 25e serves as the sandwiching surface that sandwiches the blood sensor with second holder 81b (see FIG.25).

Plate-like elastic bodies 81c are formed integrally with first holder 81a using the same material as the body of first holder 81a and have flexibility to be deformed elastically.

These two plate-like elastic bodies 81c are formed between hole 25g and connectors 49 and in the vicinity of positioning convex parts 25k, respectively. In addition, plate-like elastic bodies 81c are located around the side surfaces of the first holder, which extend parallel to the direction in which blood sensor 23 is ejected and are in contact with blood sensor 23.

Specifically, plate-like elastic bodies 81c support blood sensor 23 not in the positions w here the spindle of second holder 81b is located upstream of the inserting direction of blood sensor 23 but in the position in the ejecting direction of blood sensor 23. Here, two plate-like elastic bodies 81c are provided on both sides (lateral sides) of hole 25g formed at approximately the center of under surface 25e, and are provided so as to incline and extend to the connectors 49 side in the ejecting direction of the blood sensor.

Although the biasing force applied to blood sensor 23, resulting from the elastic deformation of plate-like elastic bodies 81c weakens than the biasing force of leaf spring 25c (see FIG.5 and FIG.6), which pushes second holder 81b against first holder 81a, it is enough to push up blood sensor 23 by touching blood sensor 23 with the extended tips. Plate-like elastic bodies 81c touch blood sensor 23 with their tips so as to reduce their contact area with blood sensor 23 By this means, a possibility to transfer blood 10 applied to blood sensor 23 to plate-like elastic bodies 81c is minimized.

In addition, these plate-like elastic bodies 81c are mounted to be placed apart from under surface 25e, from hole 25g side toward the connectors 49 side. Therefore, plate-like elastic bodies 81c do not interfere blood sensor 23 that is inserted in the sandwiching area formed with second holder 81b in puncturing section 81, so that blood sensor 23 is smoothly carried out to puncturing section 81. Second holder 81b as shown in FIG.25 has the same basic configuration as second holder 25b according to embodiment 1 except second holder 81b has plate-like elastic bodies 81d.

That is, second holder 81b faces first holder 81a as shown in FIG.26 and sandwiches blood sensor 23 to be conveyed by top surface 25v facing under surface 25e of first holder 81a, which serves as the sandwiching surface.

Second holder 81b is provided pivotably around spindle 25r as with second holder 25b of embodiment 1 and is mounted to move in the direction to contact under surface 25e and in the direction to part from under surface 25e, which is the sandwiching surface of first holder 81a.

That is, second holder 81b has tongue piece 25p inclining and projecting in the direction to part from the first holder 81a side in second holder body 25s of second holder 81b having top surface 25v, in the position closer to the base end side than a position where spindle 25 is formed, that is, in a position upstream of the conveying direction of blood sensor 23, as with second holder 25b according to the above-described embodiment 1.

This tongue piece 25p is pushed up by pressing projection 80b (corresponding to pressing projection 20b of embodiment 1) of the pushing-up section, which is gap defining section 80 provided in cover 22b, and this pushing up of tongue piece 25p allows second holder 81b to rotate around spindle 25r and move in the direction to part from first holder 81a.

Second holder 81b has plate-like elastic bodies 81d (resin leaf springs) on top surface 25v, which are elastic bodies projecting from top surface 25v serving as the sandwiching surface that sandwiches blood sensor 23 with first holder 81a.

These plate-like elastic bodies 81d are formed integrally with second holder 81b using the same material as the body of second holder 81b and have flexibility to be deformed elastically. Here, these plate-like elastic bodies 81d correspond to plate-like elastic bodies 81c formed in first holder 81a, and are formed in second holder 81b in the same manner as plate-like elastic bodies 81c. That is, two plate-like elastic bodies 81d provided in the positions facing plate-like elastic bodies 81c of first holder 81a, and support blood sensor 23.

Plate-like elastic bodies 81d support blood sensor 23 on the upper surface 25v of second holder 81b, not in the positions where spindle is located but in the position downstream of conveying direction of blood sensor 23.

Here, two plate-like elastic bodies 81d are provided on both sides (lateral sides) of hole 25m formed at approximately the center of top surface 25v, and face plate-like elastic bodies 81c so as to incline and extend, from the location near the long holes in which positioning convex parts 25k are inserted, out to the downstream of the ejecting direction.

Although the biasing force applied to blood sensor 23, resulting from elastic deformation of plate-like elastic bodies 81d weakens than the biasing force of leaf spring 25c (see FIG.26A, B and C), which pushes second holder 81b against first holder 81a, it is enough to push up blood sensor 23 by touching blood sensor 23 with their extended tips. Plate-like elastic bodies 81d contact blood sensor 23 at their tips so as to reduce the contact area with blood sensor 23. By this means, a possibility to transfer blood 10 applied to blood sensor 23 to plate-like elastic bodies 81d is minimized. As described above, also plate-like elastic bodies 81d are mounted so as to be placed apart from upper surface 25v from hole 25m side toward the downstream of the ejecting direction. Therefore, plate-like elastic bodies 81d do not interfere blood sensor 23 that is inserted in the sandwiching area formed with first holder 81a in puncturing section 81, so that blood sensor 23 is smoothly carried out to puncturing section 81.

In addition, gap defining section 85 included in blood test apparatus 83 according to embodiment 2 omits supporting pawls 20c of pushing-up section as shown in FIG.26B and FIG.26C, differently from gap defining section 20 of embodiment 1.

That is, pushing-up section 80 projects from pushing-up section body 80a provided inside cover 22b to the housing body 22a side and has pressing projection 80b that pushes up tongue piece 25p of second holder 81b of puncturing section 81 in housing body 22a (see FIG.3) when cover 22b (see FIG.3) is closed.

Pushing up by pressing projection 80b allows second holder 81b to rotate around spindle 25r and first holder 81a is placed apart from first holder 81a. Here, another configuration may be applicable where pressing projection 80b abuts on shatter 24n of cartridge 24 (see FIG.3, FIG.4 and FIG.19) mounted in housing body 20a when cover 22b is closed and pushes up shutter 24n to make sensor outlet 24a face puncturing section 81, as with pressing projection 20b of embodiment 1.

Next, operation of gap defining section 85 of blood test apparatus 83 according to embodiment 2 will be described.

FIG.26A is a cross sectional view showing the first state in which blood sensor 23 is inserted, sandwiched and fixed between first holder 81a and second holder 81b.

This state shows that puncturing section 81 can perform puncturing as with the first state of the above-described embodiment 1. That is, in blood test apparatus 83, cover 22b (see FIG.3 and FIG.4) is open and puncturing section 81 in housing body 22a is exposed outside.

In addition, second holder 81b is pressed toward first holder 81a by leaf spring 25c provided in housing body 22a and is kept parallel to first holder 81a by leaf spring 25c, as with puncturing section 25 of the above-described embodiment 1. At this time, plate-like elastic bodies 81c and 81d provided on respective sandwiching surfaces sandwiching blood sensor 23 of first holder 81a and second holder 81b resist a biasing force of leaf spring 25c and are bent. That is, plate-like elastic bodies 81c and 81d are elastically deformed and do not affect the airtightness between first holder 81a and blood sensor 23 and between second holder 81b and blood sensor 23. In this state, puncturing is performed by laser unit 26 (see FIG.21) to perform a blood test. After the blood test is completed, cover 22b is closed.

FIG.26B is a cross sectional view showing the second state. The second state shows that operation of puncturing and blood-testing by laser unit 26 are completed and cover 22b is closed.

When cover 22b is closed, pressing projection 80b of pushing-up section 80 mounted in cover 22b pushes up tongue piece 25p of second holder 81b.

When tongue piece 25p is pushed up, second holder 81b rotates around spindle 25r, is placed apart from first holder 81a and move so as to open in the outlet 22f side. Following this, blood sensor 23 is placed apart from first holder 81a and tries to move so as to incline to the outlet 22f side. When second holder 81b is placed apart from first holder 81a, plate-like elastic bodies 81c and 81d return to the original condition and move in the direction where their tips are placed apart from respective sandwiching surfaces. By this means, the tips of plate-like elastic bodies 81c and 81d press blood sensor 23 and support blood sensor 23 while blood sensor 23 is placed apart from respective first holder 81a and second holder 81b.

That is, plate-like elastic bodies 81c and 81d project from sandwiching surfaces of first holder 81a and second holder 81b in the direction to face one another, and, when second holder 81b is placed apart from first holder 81a, their projecting ends (tips) abut on blood sensor 23. Those plate-like elastic bodies 81c and 81d are located on sandwiching surfaces that sandwich blood sensor 23, along both rims parallel to the ejecting direction of blood sensor 23, and abut on the end (tip) of blood sensor 23 in the ejecting direction side of blood sensor 23.

As described above, pressing by pressing projection 80b allows second holder 81b to be placed apart from first holder 81a, so that blood sensor 23 is supported by plate-like elastic bodies 81c and 81d.

By this means, blood sensor 23 is placed apart from first holder 81a and second holder 81b and lightly supported by plate-like elastic bodies 81c and 81d. That is, plate-like elastic bodies 81c and 81d define gap 82a between first holder 81a and blood sensor 23 and define gap 82b between second holder 81b and blood sensor 23.

In second embodiment as described above, gaps 82a and 82b are defined between blood sensor 23 and both first holder 81a and second holder 81b that sandwich blood sensor 23, respectively when cover 22b is closed after puncturing is performed. Therefore, first holder 81a and second holder 81b are not stained with blood 10 applied to blood sensor 23.

FIG.26C is a cross sectional view showing the third state. This third state explains removal of blood sensor 23.

When cover 22b is closed after puncturing is performed, blood sensor 23 after puncturing is supported by plate-like elastic bodies 81c and 81d while blood sensor 23 is placed apart from both first holder 81a and second holder 81b. Therefore, as shown in FIG.26C, it is possible to eject and discard blood sensor 23 from outlet 22f without touching the location (storing section 34) of blood sensor 23, to which the blood is applied to housing 22 and other components as well as first holder 81a and second holder 81b.

In the present embodiment as described above, blood sensor 23 is supported and held by the tips of plate-like elastic bodies 81c and 81d. Therefore, blood test apparatus 83 (corresponding to blood test apparatus 21 of embodiment 1) can be used without resorting to its posture such as inclination. In addition, blood sensor 23 is lightly locked by plate-like elastic bodies 81c and 81d and can be easily pulled out without staining first holder 81a and second holder 81b with blood 10. Moreover, since blood sensor 23 after puncturing is locked by plate-like elastic bodies 81c and 81d, the blood sensor 23 does not fall even if blood sensor 23 has any postures, so that the surround of blood sensor 23 is not stained with blood. Furthermore, ease of use can be provided.

Here, spindle 25r of second holder 81b that is provided to move in the direction to contact first holder 81a and in the direction to part from first holder 81b may be located in the vicinity of the center of top surface 25v of second holder 28b, as with the alternative example of the puncturing section according to embodiment 1. In this case, supporting guide grooves 258 opening downward are formed at the center of each side surface of first holder 81a, as with first holder 252 (see FIG.15 to FIG.18). Meanwhile, in second holder 81b, spindle guide pieces 256 that rise from the center of each side rim of holder body 25s and are provided with spindle 25r at their tips, as with second holder 254 (see FIG.15 to FIG.18). Puncturing section 81 is configured by which the spindle guide pieces formed in second holder 81b are slidably arranged in the supporting guide grooves formed in first holder 81a in the direction in which the holders are contact with one another and in the direction in which the holders are placed apart from one another.. By this means, the same operation-effect as the alternative example of the puncturing section of the blood test apparatus according to embodiment 1 can be obtained, in addition to the above-described effect.

### (Embodiment 3)

The present embodiment eliminates supporting pawls 20c from the above-described embodiment 1, and employs sensor receiving member 92 mounted between first holder 25a and second holder 25b to define gaps between first holder 25a and blood sensor 23 and between second holder 25b and blood sensor 23 when blood sensor 23 is discarded.

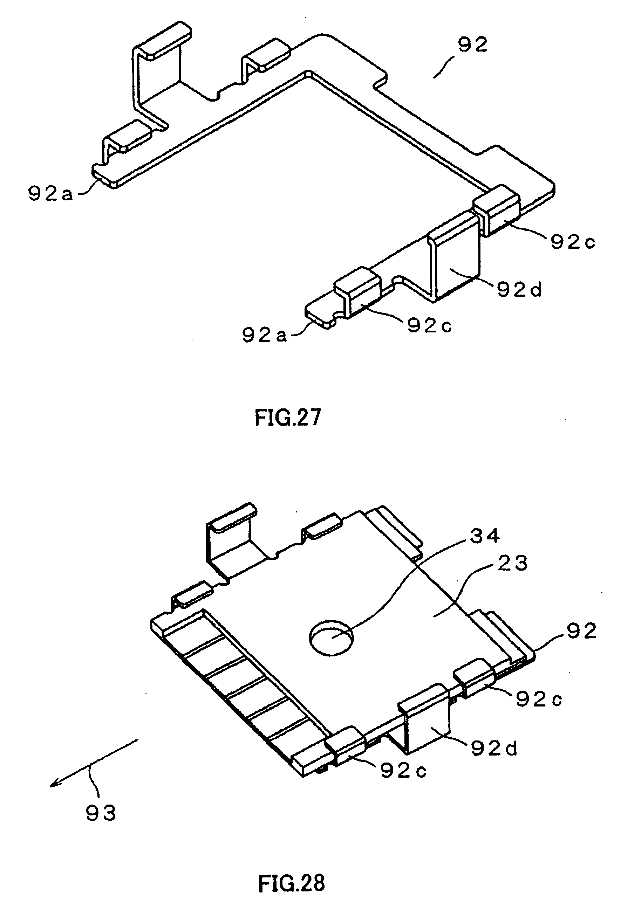

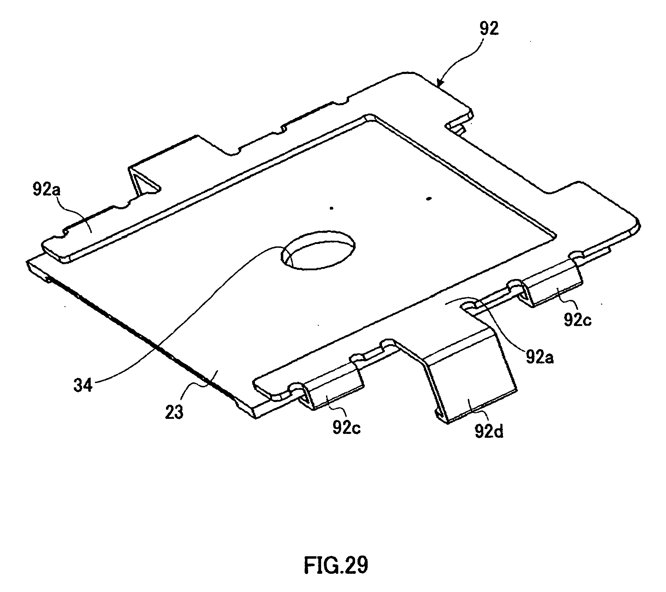

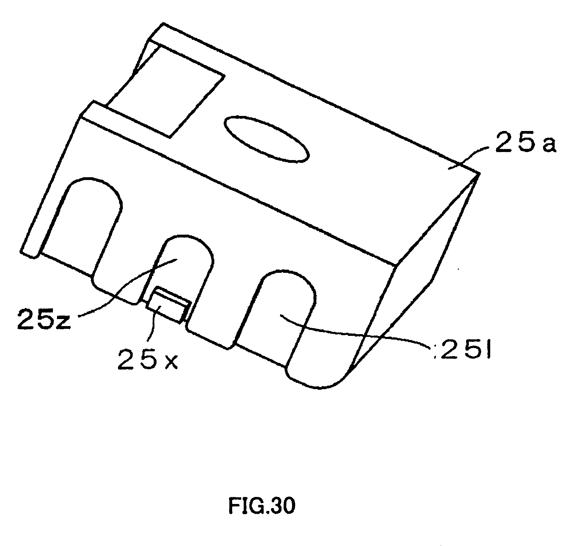

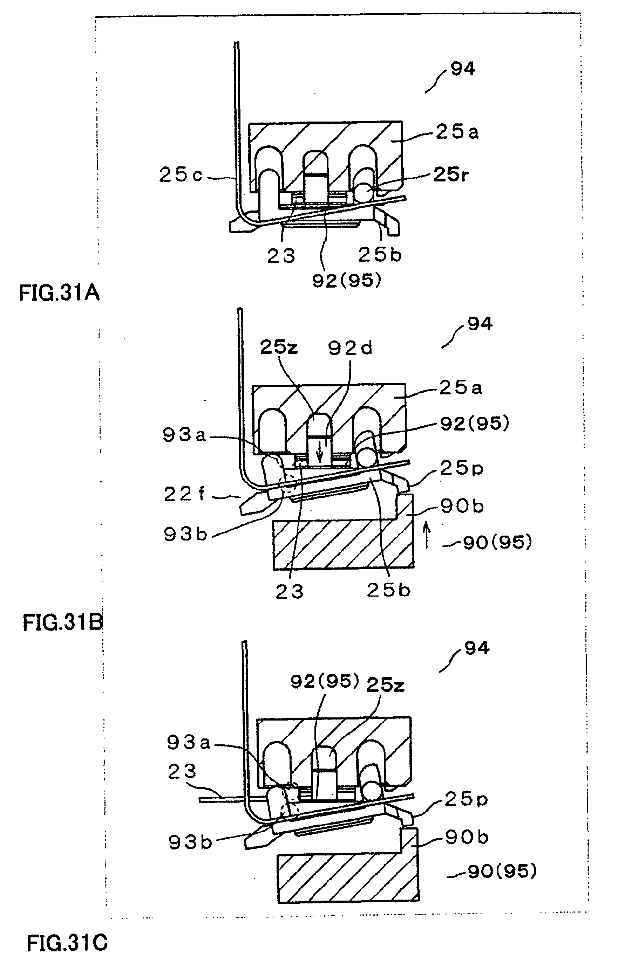

FIG.27 is an external perspective view of sensor receiving member 92 mounted between the first holder and the second holder constituting the puncturing section of the blood test apparatus according to embodiment 3. FIG.28 is a perspective view showing a state in which blood sensor 23 is inserted in sensor receiving member 92. In addition, FIG.29 is a drawing showing sensor receiving member 92 shown in FIG.28 from the back side. FIG.30 is a perspective view of first holder in which sensor receiving member 92 is suspended. In addition, FIG.31 is a cross sectional view of the primary parts explaining operation of sensor receiving member 92 according to the present embodiment. Here, FIG.31A is a cross sectional view showing the first state in which the cover is opened and puncturing section can perform puncturing in the blood test apparatus according to embodiment 3. FIG.31B is a cross sectional view showing the second state in which puncturing operation by the laser unit is completed and the cover is closed. In addition, FIG.31C is a cross sectional view showing the third state in which sensor 23 is being removed.

In blood test apparatus 94 (see FIG.31) according to this embodiment 3 has a configuration including gap defining member 95 configured by pressing projection 90 and sensor receiving member 92, which places blood sensor 23 apart from both first holder 25a and second holder 25b provided above and below when at least one of first holder 25a and second holder 25b is rotated to be spaced from one another. Here, the same components as in embodiment 1 will be assigned the same reference numerals and detailed description will be omitted.

Blood test apparatus 94 according to embodiment 3, as shown FIG.31A, has first holder 25a, and second holder 25b that is provided below first holder 25a to face first holder 25a and that rotates around spindle 25r to move in the direction to contact first holder 25a and in the direction to part from first holder 25a.

Second holder 25b is pivotably supported in the housing body (not shown) side through spindle 25r and is biased toward first holder 25a by leaf spring 25c. Here, second holder 25b is pressed by pressing projection 90b of cover 22b to rotate and move in the direction to part from first holder 25a.

Sensor receiving member 92 as shown in FIG.31A is mounted between first holder 25a and second holder 25b, and blood sensor 23 is sandwiched between first holder 25a and second holder 25b while being held by sensor receiving member 92.

Sensor receiving member 29 is suspended movably in the direction to contact first holder 25a and in the direction to part from first holder 25a.

The material of sensor receiving member 92 is metal, and has a U-shaped (plane view) supporting bottom plate that opens in the direction in which blood sensor 23 (see FIG.28) is inserted. Blood sensor 23 is placed on this supporting bottom plate. Two sensor holding sections 92c and suspending section 92d provided between these sensor holding sections 92c are provided on each of parallel sections 92a that are placed apart from and parallel to one another on this U-shaped supporting bottom plate.

Sensor holding sections 92c rise outer rims of both parallel sections 92c, and their rising ends are bent so as to face the supporting bottom end.

As shown in FIG.28, when blood sensor 23 is placed on the supporting bottom plate, from the base end side of both parallel sections 92a, the rising ends are arranged so as to sandwich blood sensor 23 above and below in cooperation with supporting bottom plate.

Blood sensor 23 is slidably supported on the supporting bottom plate by four sensor holding sections 92c.

Here, sensor receiving member 92 receives all or part of the surround of the surface of blood sensor 23 extending in the direction parallel to its ejecting direction. As shown in FIG.29, this supporting bottom plate is a part that is placed apart from storing section 34 for storing blood 10 formed at approximately the center of blood sensor 23, and holds blood sensor 23. In addition, the supporting bottom supports and holds all or part of both sides of blood sensor 23 along the inserting direction.

Blood sensor 23 is punctured by laser unit 26 while being placed on the supporting bottom plate of sensor receiving member 92. Sensor receiving member 92 has a U-shape and supports the surround parts (both rims) of blood sensor 23, which are placed apart from storing section 34 at the center part. Therefore, blood 10 introduced into storing section 34 is not contact with sensor receiving member 92. After puncturing is performed, blood sensor 23 is ejected in ejecting direction 93.

Suspending sections 92d rises from the outsides of both parallel sections 92a, respectively, and their tips are slidably engaged with first holder 25a in the direction to contact first holder 25a and in the direction to part from first holder 25a.

As shown in FIG.30, suspending sections 92d are slidably provided in guide groove parts 25z that are formed on both side walls of first holder 25a, respectively and that extend orthogonal to the bottom surface as the sandwiching surface. Pawls 25x are provided at the ends of those guide groove parts in the bottom surface side and are engaged with the tips of suspending sections 92d when suspending sections 92d move to the end of the guide groove parts in the bottom surface side. By this means, the supporting bottom plate of sensor receiving member 92 can be suspend between and parallel to first holder 25a and second holder 25b, in a position apart from the bottom surface, which is the sandwiching surface of first holder 25a at a predetermined distance.

As described above, sensor receiving member 92 is slidably suspended in the direction to contact first holder 25a and in the direction to part from first holder 25a.

Next, operation of the gap defining section of the present embodiment will be described.

As shown in FIG.31 A, in the state in which cover 22b (see FIG.3 and FIG.4) is opened from housing body 22a, second holder 25b is biased toward first holder 25a by leaf spring 25c. Therefore, sensor 23 placed on sensor receiving member 92 is sandwiched and fixed between first holder 25a and second holder 25b that is biased in the direction in which the second holder 25b is attached firmly to first holder 25a and which is parallel to first holder 25a. In this state, blood sensor 23 is punctured by laser unit 26 (see FIG.20).

When the puncturing operation by laser unit 26 is completed and cover 22b is closed, pressing projection 90b of pressing section 90 mounted in cover 22b pushes up tongue piece 25p formed in second holder 25b. This pressing-up of tongue piece 25p by pressing projection 90b allows second holder 25b to rotate around spindle 25r, be placed apart from the sandwiching surface (i.e. bottom surface) of first holder 25a and incline so as to open its end in the outlet 22 side (see the second state shown in FIG.31B).

When puncturing section 25 opens in outlet 22f side of sandwiching area formed by first holder 25a and second holder 25b, blood sensor 23 is suspended by sensor receiving member 92 in first holder 25a. At this time, sensor receiving member 92 is engaged with first holder 25a through suspending sections 92d that can slide on first holder 25a, so that gap 93 a is defined between first holder 25a and sensor 23 by gravity. In addition, since second holder 25b opens, gap 93b is also defined between sensor 23 and second holder 25b.

That is, when first holder 25a and second holder 25b are placed apart from one another by pushing-up of pressing projection 90b, sensor receiving member 92 between these holders 25a and 25b is placed apart from first holder 25a by its own weight, so that gap 93a is defined between sensor receiving member 92 and first holder 25a. In addition, rotated second holder 25b is located lower than the height of sensor receiving member 92 that comes down by its own weight and is placed apart from first holder 25a, so that gap 93b is defined between sensor receiving member 92 and second holder 25b.

As described above, in the blood test apparatus according to embodiment 3, sensor receiving member 92 holds blood sensor 23 in the position where blood sensor 23 is placed apart from first holder 25a and second holder 25b by gaps 93a and 93b, respectively. After puncturing is performed, first holder 25a and second holder 25b are not stained with blood 10 applied to blood sensor 23.

In addition, as shown in FIG.31C, when blood sensor 23 is ejected from the outlet 22f side, pressing projection 90b pushes up tongue piece 25p, so that first holder 25a and second holder 25b are placed apart from one another.

Used blood sensor 23 can be slid through outlet 22f and pulled out from sensor receiving member 92 that holds blood sensor 23 while blood sensor 23 is placed apart from both holder 25a and 25b that are placed apart from one another. At this time, blood sensor 23 can be pulled out without staining first holder 25a and second holder 25b with blood 10 applied to blood sensor 23. Here, the supporting bottom plate of sensor receiving member 92 is formed as a U-shape that opens toward the outlet. Therefore, when discarding punctured blood sensor 23 from housing 22, the user can insert his/her fingers between both parallel sections of the supporting bottom plate through outlet 22f, pick up, in the vertical direction, the end (tip) of blood sensor 23 in the outlet side and easily pull out blood sensor 23.

In addition, in embodiment 3, when being placed on sensor receiving member 92, blood sensor 23 is held by sensor holding sections 92c. Therefore, when being punctured or discarded, blood sensor 23 can be prevented from laterally displacing from the holding position of puncturing section 25. Here, sensor receiving member 92 may be combined with plate-like elastic members 81c and 81d described in the above embodiment 2. Therefore, the sensor receiving member is not spaced from first holder 25a by its own weight but is placed apart from the sandwiching surface of first holder 25a by resilience of the plate-like elastic bodies projecting from the first holder25a side. Therefore, the same effect as the above-described embodiment 2 such that puncturing and blood-testing can be performed without limiting orientation in use of the blood test apparatus itself can be obtained at the same time.

The present invention claims priority based on Japanese Patent Application No.2007-202488, filed on August 3, 2007.

### Industrial Applicability

In the blood test apparatus according to the present invention, the blood sensor can be discarded after puncturing without staining the puncturing section with blood, so that it is useful.

## Claims

1. A blood test apparatus that takes blood exuding from punctured skin into a blood sensor to analyze components of the blood, the blood test apparatus comprising:
a first holder and a second holder that sandwich the blood sensor, at least one of the first holder and the second holder is provided movably in a direction in contact with the other holder and in a direction to part from the other holder; and
a gap defining section that moves at least the one holder, so that the first holder and the second holder are placed apart from one another, that supports the blood sensor apart from both the first holder and the second holder, and that defines gaps between the blood sensor and the first holder and between the blood sensor and the second holder.

2. The blood test apparatus according to claim 1, further comprising:
a housing body including the first holder and the second holder and having an opening facing at least one of the first holder and the second holder; and
a cover that can open and close, and that covers the opening of the housing body,
wherein the gap defining section is provided in the cover, and, when the cover covers the opening of the housing body, the gap defining section moves the one holder to support the blood sensor such that the blood sensor is placed apart from both the first holder and the second holder.

3. The blood test apparatus according to claim 1, wherein:
the first holder is fixed to a housing;
the second holder is pivotably mounted in the housing, around a spindle provided at one end side of a sandwiching surface facing the first holder and moves by rotation, such that the distance from the first holder gradually increases toward the other end side of the sandwiching surface;
the gap defining section includes:
a pressing section that presses the one end side of the second holder and rotates the second holder;
supporting pawls that project from the other end side of the second holder when the second holder rotates, and support the blood sensor between the first holder and the second holder.

4. The blood test apparatus according to claim 1, wherein:
the first holder is fixed to a housing;
the second holder is mounted pivotably around a spindle provided in the vicinity of a center of a sandwiching surface facing the first holder, inclines to the first holder by rotation and moves such that the distance from the first holder gradually increases from the one end side toward the other end side of the sandwiching surface; and
the gap defining section includes:
a pressing section that presses the one end side of the second holder and rotates the second holder; and
supporting pawls that project from the other end side of the second holder when the second holder rotates and support the blood sensor between the first holder and the second holder.

5. The blood test apparatus according to claim 3, wherein the supporting pawls abut on both sides of an end, corresponding to the other end side of the second holder, of the blood sensor and support the blood sensor at the both sides.

6. The blood test apparatus according to claim 1, wherein the gap defining section projects from a sandwiching surface of each of the first holder and the second holder in the direction facing one another and has elastic bodies having projecting ends that abut on the blood sensor.

7. The blood test apparatus according to claim 6, wherein the elastic bodies are arranged along rims of the sandwiching surface that sandwiches the blood sensor, parallel to an ejecting direction of the blood sensor, and abut on an end of the blood sensor in the ejecting direction side.

8. The blood test apparatus according to claim 1, wherein:
the first holder and the second holder face to one another above and below; and
the gap defining section includes a sensor receiving member that is suspended between the first holder and the second holder arranged above and below such that sensor receiving member is slidably suspended from one of the first holder and the second holder, and the blood sensor is placed on the sensor receiving member.

9. The blood test apparatus according to claim 8, wherein the sensor receiving member receives the blood sensor at rims of the blood sensor extending parallel to an ejecting direction of the blood sensor.

10. The blood test apparatus according to claim 1, wherein:
the blood sensor has a storing section that penetrates the blood sensor in the direction in which the blood sensor is sandwiched between the first holder and the second holder and that stores therein the blood; and
the blood test apparatus further comprising:
a cartridge that stacks and stores blood sensors;
a conveying section that separates the blood sensors from the cartridge one by one and conveys the blood sensor between the first holder and the second holder; and
a puncturing section that penetrates the storing section of the blood sensor conveyed between the first holder and the second holder and punctures skin.

11. The blood test apparatus according to claim 9, wherein:
a projection is provided on a sandwiching surface of one of the first holder and the second holder, the projection engaging with a hole formed on a sandwiching surface of the other of the first holder and the second holder; and
the projection limits a sandwiched position of the blood sensor conveyed between the first holder and the second holder by the conveying section.

12. A blood test method, using a blood test apparatus according to claim 1, for taking blood exuding from punctured skin into a blood sensor to analyze components of the blood, comprising:
taking the blood into the blood sensor while the blood sensor is sandwiched between a first holder and a second holder; and
forming gaps between the blood sensor and the first holder and between the blood senor and the second holder by defining a space between the blood sensor into which the blood is taken and both the first holder and the second holder sandwiching the blood sensor, by a gap defining section.

## Patentansprüche

1. Blutprobenvorrichtung zum Entnehmen von Blut, das aus punktierter Haut austritt, in einen Blutsensor zum Analysieren von Bestandteilen des Blutes, wobei die Blutprobenvorrichtung umfasst:
einen ersten Halter und einen zweiten Halter, die den Blutsensor einschließen, wobei der erste Halter oder/und der zweite Halter in einer Richtung, in der er/sie mit dem anderen Halter in Kontakt kommt/kommen, und in einer Richtung bewegt werden kann/können, in der er/sie sich von dem anderen Halter trennt/trennen; und
einen Zwischenraum-Herstellungsabschnitt, der wenigstens den einen Halter so bewegt, dass der erste Halter und der zweite Halter voneinander getrennt positioniert werden, der den Blutsensor von dem ersten Halter sowie dem zweiten Halter getrennt hält und der Zwischenräume zwischen dem Blutsensor und dem ersten Halter sowie zwischen dem Blutsensor und dem zweiten Halter herstellt.

2. Blutprobenvorrichtung nach Anspruch 1, die des Weiteren umfasst:
einen Aufnahmekörper, der den ersten Halter sowie den zweiten Halter enthält und eine Öffnung aufweist, die dem ersten Halter oder/und dem zweiten Halter zugewandt ist; und
eine Abdeckung, die geöffnet und geschlossen werden kann und die die Öffnung des Aufnahmekörpers abdeckt,
wobei der Zwischenraum-Herstellungsabschnitt in der Abdeckung vorhanden ist, und, wenn die Abdeckung die Öffnung des Aufnahmekörpers abdeckt, der Zwischenraum-Herstellungsabschnitt den einen Halter so bewegt, dass er den Blutsensor so trägt, dass der Blutsensor sowohl von dem ersten Halter als auch dem zweiten Halter getrennt positioniert ist.

3. Blutprobenvorrichtung nach Anspruch 1, wobei:
der erste Halter an einem Gehäuse befestigt ist;
der zweite Halter in dem Gehäuse um eine Spindel herum schwenkbar angebracht ist, die an einer Endseite einer einschließenden Fläche vorhanden ist, die dem ersten Halter zugewandt ist und sich durch Drehung bewegt, so dass der Abstand von dem ersten Halter zur anderen Endseite der einschließenden Fläche hin allmählich zunimmt;
und der Zwischenraum-Herstellungsabschnitt enthält:
einen Drückabschnitt, der auf die eine Endseite des zweiten Halters drückt und den zweiten Halter dreht;
Halteklauen, die von der anderen Endseite des zweiten Halters vorstehen, wenn sich der zweite Halter dreht, und die den Blutsensor zwischen dem ersten Halter und dem zweiten Halter halten.

4. Blutprobenvorrichtung nach Anspruch 1, wobei:
der erste Halter an einem Gehäuse befestigt ist;
der zweite Halter um eine Spindel herum schwenkbar angebracht ist, die in der Nähe einer Mitte einer dem ersten Halter zugewandten einschließenden Fläche vorhanden ist,
sich durch Drehung zu dem ersten Halter neigt und sich so bewegt, dass der Abstand von dem ersten Halter von der einen Endseite zu der anderen Endseite der einschließenden Fläche hin allmählich zunimmt; und
der Zwischenraum-Herstellungsabschnitt enthält:
einen Drückabschnitt, der auf die eine Endseite des zweiten Halters drückt und den zweiten Halter dreht;
Halteklauen, die von der anderen Endseite des zweiten Halters vorstehen, wenn sich der zweite Halter dreht, und die den Blutsensor zwischen dem ersten Halter und dem zweiten Halter halten.

5. Blutprobenvorrichtung nach Anspruch 3, wobei die Halteklauen an beiden Seiten eines der anderen Endseite des zweiten Halters entsprechenden Endes des Blutsensors anliegen und den Blutsensor an den beiden Seiten halten.

6. Blutprobenvorrichtung nach Anspruch 1, wobei der Zwischenraum-Herstellungsabschnitt von einer einschließenden Fläche des ersten und des zweiten Halters in der Richtung vorsteht, in der sie einander zugewandt sind, und er elastische Körper mit vorstehenden Enden aufweist, die an dem Blutsensor anliegen.

7. Blutprobenvorrichtung nach Anspruch 6, wobei die elastischen Körper an Rändern der einschließenden Fläche, die den Blutsensor einschließt, parallel zu einer Ausstoßrichtung des Blutsensors angeordnet sind und an einem Ende des Blutsensors an der Seite in der Ausstoßrichtung anliegen.

8. Blutprobenvorrichtung nach Anspruch 1, wobei:
der erste Halter und der zweite Halter einander oberhalb und unterhalb zugewandt sind; und
der Zwischenraum-Herstellungsabschnitt ein Sensor-Aufnahmeelement enthält, das zwischen dem ersten Halter und dem zweiten Halter, die oberhalb und unterhalb angeordnet sind, so aufgehängt ist, dass das Sensor-Aufnahmeelement verschiebbar an dem ersten Halter oder dem zweiten Halter aufgehängt ist, und der Blutsensor an dem Sensor-Aufnahmeelement positioniert ist.

9. Blutprobenvorrichtung nach Anspruch 8, wobei das Sensor-Aufnahmeelement den Blutsensor an Rändern des Blutsensors aufnimmt, die parallel zu einer Ausstoßrichtung des Blutsensors verlaufen.

10. Blutprobenvorrichtung nach Anspruch 1, wobei:
der Blutsensor einen Aufbewahrungsabschnitt aufweist, der durch den Blutsensor in der Richtung hindurch verläuft, in der der Blutsensor zwischen dem ersten Halter und dem zweiten Halter eingeschlossen ist, und in dem das Blut aufbewahrt wird; und
die Blutprobenvorrichtung des Weiteren umfasst:
eine Kartusche, in der Blutsensoren übereinander angeordnet und aufbewahrt sind;
einen Transportabschnitt, der die Blutsensoren nacheinander aus der Kartusche entfernt und den Blutsensor zwischen dem ersten Halter und dem zweiten Halter transportiert; und
einen Punktionsabschnitt, der sich durch den Aufbewahrungsabschnitt des zwischen dem ersten Halter und dem zweiten Halter transportieren Blutsensors hindurch erstreckt und Haut punktiert.

11. Blutprobenvorrichtung nach Anspruch 9, wobei:
ein Vorsprung an einer einschließenden Fläche des ersten Halters oder des zweiten Halters vorhanden ist, der Vorsprung mit einem Loch in Eingriff ist, das an einer einschließenden Fläche des anderen von dem ersten Halter und dem zweiten Halter ausgebildet ist, und
der Vorsprung eine eingeschlossene Position des durch den Transportabschnitt zwischen dem ersten Halter und dem zweiten Halter transportierten Blutsensors begrenzt.

12. Blutprobenverfahren, bei dem eine Blutprobenvorrichtung nach Anspruch 1 eingesetzt wird, zum Entnehmen von aus punktierter Haut austretendem Blut in einen Blutsensor und zum Analysieren von Bestandteilen des Blutes, wobei das Verfahren umfasst:
Entnehmen des Blutes in den Blutsensor, während der Blutsensor zwischen einem ersten Halter und einem zweiter Halter eingeschlossen ist; und
Ausbilden von Zwischenräumen zwischen dem Blutsensor und dem ersten Halter sowie zwischen dem Blutsensor und dem zweiten Halter durch Herstellen eines Raums zwischen dem Blutsensor, in den das Blut entnommen wird, und sowohl dem ersten Halter als auch dem zweiten Halter, die den Blutsensor einschließen, mittels eines Zwischenraum-Herstellungsabschnitts.

## Revendications

1. Appareil d'analyse du sang qui prélève du sang exsudant d'une peau ponctionnée dans un capteur sanguin pour analyser des composants du sang, l'appareil d'analyse du sang comprenant :
un premier support et un deuxième support entre lesquels est intercalé le capteur sanguin, au moins un support parmi le premier support et le deuxième support étant agencé de façon mobile dans une direction pour entrer en contact avec l'autre support et dans une direction pour s'écarter de l'autre support ; et
une section définissant un intervalle qui déplace au moins ledit support de telle sorte que le premier support et le deuxième support sont écartés l'un de l'autre, qui supporte le capteur sanguin à l'écart des premier et deuxième supports, et qui définit des intervalles entre le capteur sanguin et le premier support, et entre le capteur sanguin et le deuxième support.

2. Appareil d'analyse du sang selon la revendication 1, comprenant en outre :
un corps de boîtier comprenant le premier support et le deuxième support et comportant une ouverture qui fait face à au moins un des premier et deuxième supports ; et
un couvercle qui peut s'ouvrir et se refermer et qui couvre l'ouverture du corps de boîtier,
dans lequel la section définissant un intervalle est pourvue dans le couvercle, et lorsque le couvercle couvre l'ouverture du corps de boîtier, la section définissant un intervalle déplace ledit support qui supporte le capteur sanguin de telle sorte que le capteur sanguin est positionné à l'écart des premier et deuxième supports.

3. Appareil d'analyse du sang selon la revendication 1, dans lequel :
le premier support est fixé à un boîtier ;
le deuxième support est monté de manière pivotante dans le boîtier, autour d'un axe pourvu à un côté d'extrémité d'une surface d'intercalage faisant face au premier support, et se déplace par rotation de telle sorte que la distance depuis le premier support augmente graduellement vers l'autre côté d'extrémité de la surface d'intercalage ;
la section définissant un intervalle comprend :
une section de compression qui comprime ledit un côté d'extrémité du deuxième support et fait tourner le deuxième support ;
des cliquets de support qui font saillie de l'autre côté d'extrémité du deuxième support lorsque le deuxième support tourne, et supportent le capteur sanguin entre le premier support et le deuxième support.

4. Appareil d'analyse du sang selon la revendication 1, dans lequel :
le premier support est fixé à un boîtier ;
le deuxième support est monté de façon pivotante autour d'un axe fourni à proximité d'un centre d'une surface d'intercalage faisant face au premier support, s'incline vers le premier support par rotation, et se déplace de telle sorte que la distance depuis le premier support augmente graduellement depuis ledit côté d'extrémité vers l'autre côté d'extrémité de la surface d'intercalage ; et
la section définissant un intervalle comprend :
une section de compression qui comprime ledit un côté d'extrémité du deuxième support et fait tourner le deuxième support ; et
des cliquets de support qui font saillie depuis l'autre côté d'extrémité du deuxième support lorsque le deuxième support tourne et supportent le capteur sanguin entre le premier support et le deuxième support.

5. Appareil d'analyse du sang selon la revendication 3, dans lequel les cliquets de support viennent à butée des deux côtés d'une extrémité du capteur sanguin, correspondant à l'autre côté d'extrémité du deuxième support, et supportent le capteur sanguin des deux côtés.

6. Appareil d'analyse du sang selon la revendication 1, dans lequel la section définissant un intervalle s'étend depuis une surface d'intercalage de chacun des premier et deuxième supports dans la direction où ils se font face, et comporte des corps élastiques ayant des extrémités saillantes qui viennent à butée contre le capteur sanguin.

7. Appareil d'analyse du sang selon la revendication 6, dans lequel les corps élastiques sont agencés le long de bords de la surface d'intercalage entre laquelle est intercalé le capteur sanguin, parallèlement à une direction d'éjection du capteur sanguin, et viennent à butée contre une extrémité du capteur sanguin du côté de la direction d'éjection.

8. Appareil d'analyse du sang selon la revendication 1, dans lequel :
le premier support et le deuxième support se font face au-dessus et en dessous ; et
la section définissant un intervalle comprend un élément recevant le capteur qui est suspendu entre les premier et deuxième supports agencés au-dessus et en dessous de telle sorte que l'élément recevant le capteur est suspendu de façon coulissante à l'un des premier et deuxième supports, et le capteur sanguin est placé sur l'élément recevant le capteur.

9. Appareil d'analyse du sang selon la revendication 8, dans lequel l'élément recevant le capteur reçoit le capteur sanguin à des bords du capteur sanguin qui s'étendent parallèlement à une direction d'éjection du capteur sanguin.

10. Appareil d'analyse du sang selon la revendication 1, dans lequel :
le capteur sanguin présente une section de stockage qui pénètre dans le capteur sanguin dans la direction selon laquelle le capteur sanguin est intercalé entre les premier et deuxième supports et qui y stocke le sang ; et
l'appareil d'analyse du sang comprenant en outre :
une cartouche qui empile et stocke des capteurs sanguins ;
une section de transport qui sépare les capteurs sanguins un par un de la cartouche et transporte le capteur sanguin entre le premier support et le deuxième support ; et
une section de ponction qui pénètre dans la section de stockage du capteur sanguin transporté entre le premier support et le deuxième support et ponctionne la peau.

11. Appareil d'analyse du sang selon la revendication 9, dans lequel :
une projection est pourvue sur une surface d'intercalage d'un support parmi les premier et deuxième supports, la projection s'engageant dans un trou formé dans une surface d'intercalage de l'autre desdits premier et deuxième supports ; et
la projection délimite une position intercalée du capteur sanguin transporté entre le premier support et le deuxième support par la section de transport.

12. Procédé d'analyse du sang utilisant un appareil d'analyse du sang selon la revendication 1 pour prélever du sang exsudant d'une peau ponctionnée dans un capteur sanguin pour analyser des composants du sang, comprenant :
le prélèvement du sang dans le capteur sanguin pendant que le capteur sanguin est intercalé entre un premier support et un deuxième support ; et
la formation d'intervalles entre le capteur sanguin et le premier support et entre le capteur sanguin et le deuxième support en définissant un espace entre le capteur sanguin dans lequel le sang est prélevé et les premier et deuxième supports entre lesquels est intercalé le capteur sanguin, par une section définissant un intervalle.
